# EUROPEAN PATENT SPECIFICATION

(11) **EP 1 874 954 B1**
(45) Date of publication and mention of the grant of the patent: **27.12.2017**
(21) Application number: 06751967.8
(22) Date of filing: 27.04.2006
(51) Int. Cl.: A61F 2/915, A61B 17/88, A61F 2/30, A61F 2/91, A61F 2/44, A61F 2/46

(54) **EXPANDABLE SUPPORT DEVICE**
EXPANDIERBARE STÜTZVORRICHTUNG
DISPOSITIF SUPPORT EXPANSIBLE

(30) Priority: 27.04.2005 US 675512 P; 11.11.2005 US 735718 P
(43) Date of publication of application: 09.01.2008
(73) Proprietor: Stout Medical Group, L.P., Quakertown, PA 18951 (US)
(72) Inventor: GREENHALGH, Skott, E., Gladwyne, PA 19035 (US); ROMANO, John, Paul, Chalfont, PA 18914 (US); IGOE, Michael, P., Windham, NH 03087 (US)
(74) Representative: J A Kemp
(86) International application number: PCT/US2006/016554
(87) International publication number: WO 2006/116761

(56) References cited:
- EP-A1- 0 758 541
- WO-A1-00/44319
- WO-A2-00/30523
- WO-A2-00/44321
- US-A- 5 171 278
- US-A- 5 556 413
- US-A- 5 827 321
- US-A1- 2004 111 108
- US-A1- 2004 153 146
- US-B1- 6 551 342
- DATABASE WPI Week 198004 Thomson Scientific, London, GB; AN 1980-A8866C XP002690114, -& SU 662 082 A1 (TARTUS UNIV) 15 May 1979 (1979-05-15)

## Description

### FIELD OF THE INVENTION

This invention relates to expandable support devices for biological implantation and methods of using the same. More specifically, the expandable support devices can be used to treat vertebral, vascular, and valvular disorders.

### BACKGROUND OF THE INVENTION

This invention relates to devices for providing support for biological tissue, for example to repair spinal compression fractures, and methods of using the same.

Vertebroplasty is an image-guided, minimally invasive, nonsurgical therapy used to strengthen a broken vertebra that has been weakened by disease, such as osteoporosis or cancer. Vertebroplasty is often used to treat compression fractures, such as those caused by osteoporosis, cancer, or stress.

Vertebroplasty is often performed on patients too elderly or frail to tolerate open spinal surgery, or with bones too weak for surgical spinal repair. Patients with vertebral damage due to a malignant tumor may sometimes benefit from vertebroplasty. The procedure can also be used in younger patients whose osteoporosis is caused by long-term steroid treatment or a metabolic disorder.

Vertebroplasty can increase the patient's functional abilities, allow a return to the previous level of activity, and prevent further vertebral collapse. Vertebroplasty attempts to also alleviate the pain caused by a compression fracture.

Vertebroplasty is often accomplished by injecting an orthopedic cement mixture through a needle into the fractured bone. The cement mixture can leak from the bone, potentially entering a dangerous location such as the spinal canal. The cement mixture, which is naturally viscous, is difficult to inject through small diameter needles, and thus many practitioners choose to "thin out" the cement mixture to improve cement injection, which ultimately exacerbates the leakage problems. The flow of the cement liquid also naturally follows the path of least resistance once it enters the bone - naturally along the cracks formed during the compression fracture. This further exacerbates the leakage.

The mixture also fills or substantially fills the cavity of the compression fracture and is limited to certain chemical composition, thereby limiting the amount of otherwise beneficial compounds that can be added to the fracture zone to improve healing. Further, a balloon must first be inserted in the compression fracture and the vertebra must be expanded before the cement is injected into the newly formed space.

A vertebroplasty device and method that eliminates or reduces the risks and complexity of the existing art is desired. A vertebroplasty device and method that is not based on injecting a liquid directly into the compression fracture zone is desired.

Furthermore, completely or partially blocked blood vessels can be repaired by angioplasty and stenting. Angioplasty entails the reconstruction or recanalization of the vessel. A common method of angioplasty includes deploying a balloon to the blockage and inflating the balloon to push the blockage out of the lumen of the vessel. Often a stent is deployed when the balloon is inflated. The stent provides structural support and can deploy drugs locally to the blockage site. During inflation of the balloon, blood flow through the vessel is partially or completely interrupted.

Aneurysms are often treated by deploying solid, liquid or gel agents to act as an embolism in the aneurysm. The liquids and gels can leak from the aneurysm during regular blood flow. The solids, often coils, are usually soft and undersized, so several coils must be deployed in a single aneurysm to fill the aneurysm. Further more, deploying solids into the weak-walled aneurysm increases the risk of rupturing the aneurysm. Also, certain configurations of aneurysms, such as those with large necks, or not discernable necks at all, are not good candidates for coil or other solid embolization since there is no natural neck to retain the implanted emboli.

Valvular disorders, such as valvular stenosis, or other valvular insufficiencies can be treated by removing the existing leaflets in the valve and implanting an artificial valve. This procedure is usually performed as an "open" procedure, during which the patient undergoes severe trauma, such as a broken sternum and a large wound, that is extemporaneous to the replacement of the valve.

WO 00/44319 A1 discloses an expanding spacer. The spacer initially comprises a structure having a narrow diameter. When the spacer is expanded, the diameter increases. The diameter of the spacer increases at the expense of the length of the spacer, which is shortened. The spacer is formed of a hollow tube having a plurality of axial slits formed on its surface. The slits are arranged in pairs of parallel slits, each pair defining a spike, which spike is formed when the material between the slits is folded perpendicular to the slits. When the tube is compressed, the spikes fold out in the shape of an inverted "V".

WO 00/44321 A2 discloses an apparatus for controlling the deformation of an implant during deployment thereof. A force application mechanism applies deforming force to the implant, by motion of a force applicator against the implant. A restraint element positioning mechanism positions a restraining element such that the deformation of the implant is controlled by restraint of the restraining element on allowable deformation. A synchronizer synchronizes the motion of the restraining element and the force applicator so as to achieve a desired deformation of the implant.

US 2004/111108 A1 discloses an angioplasty balloon including a non-deployable stent to prevent or reduce the potential for slippage of the inflated balloon with respect to the vessel wall being treated. The balloon includes a non-deployable stent that is adapted to be secured to the balloon or angioplasty balloon catheter. The stent has a proximal end, a distal end, and at least one extension section, at least one set of serpentine rings and at least one set of elongation links that allow expansion of the strut to accommodate the inflation of the balloon. The stent is made of a material so that the stent collapses upon deflation of the balloon.

US 5 556 413 A discloses a stent, adapted to be inserted within a body lumen, and designed to expand and lock in an enlarged diameter form. The stent is designed so that the central portion of the stent body can be reversibly expanded at a selected site within a vessel lumen. Stent deployment can be accomplished by means of a two-stage process which allows the physician to abort the procedure if desired or if a complication develops. In the first stage, the body of the stent is expanded and then evaluated for such criteria as location relative to the stenosis and size relative to the vessel in which it resides. At this stage expansion is reversible and the decision to implant the stent can be aborted. The second stage expands and locks the ends of the stent in place so that the stent remains permanently implanted.

SU 662 082 A1 discloses a fixative for treating tubular bone fractures.

WO 00/30523 A2 discloses a system for opening and temporarily supporting a section of a generally tubular organ. The system includes a dilation catheter, which has an integrally connected shape memory catheter tip. The shape memory catheter tip is made of a shape memory alloy. The shape memory catheter tip assumes a first shape at a first temperature and a second shape at a second temperature. The shape memory catheter tip is inserted into the body of the patient, while being in a narrow shape and expands within the body of the patient.

US 6 551 342 B1 discloses an intravascular filter device for use in capturing debris which may occur as a result of an intravascular procedure. The filter device comprises: an elongate member having a first end and a second end, the second end extending exterior of the vasculature; and a body including a first section defining a first end portion, a second section defining a mid-portion, a third section defining a second end portion, the first section, the second section and the third section form a conically tapering filter, the body includes an open first end and a substantially closed second end. The body has a longitudinal axis defined by a sidewall, the body being attached to the second end of the elongate member, and the elongate member extending from the open first end in a parallel relationship with the longitudinal axis. The mid-portion further comprises three ring members adjacently configured end to end, each ring member having a plurality of first members in an alternating V-pattern to form sixteen apices defining each a ring proximal end and a ring distal end.

### SUMMARY OF THE INVENTION

According to the present invention there is provided the expandable support device of claim 1.

Additional aspects of the invention are set out in the dependent claims.

An expandable support device is disclosed herein. The expandable support device can be used to treat orthopedic (e.g., vertebral), vascular and/or valvular disorders. Examples include treating compression fractures in the spine, long bone fractures, spinal fusion, atherosclerosis, valvular stenosis, and aneurysms.

The expandable support device can be configured to expand as a "reverse" stent: expanding radially when compressed longitudinally. The expandable support device can be deployed as a reverse stent between bones, in a bone, in a vessel, in an aneurysm across a valve, or combinations thereof

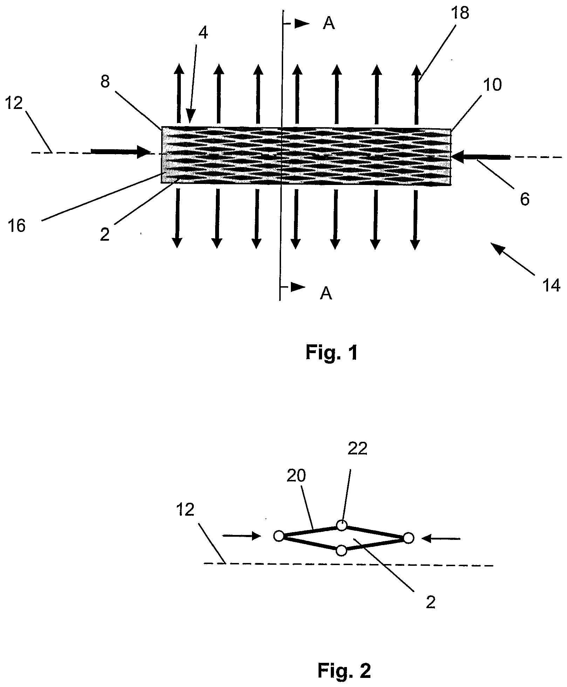

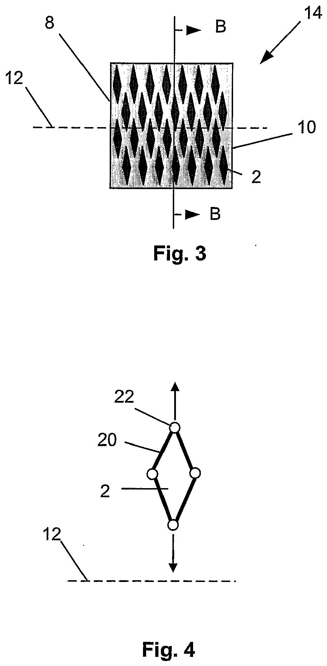

The expandable support device can be a stent. The stent can be, for example, a reverse stent. The reverse stent can be configured to radially expand (e.g., open) as the stent is longitudinally compressed. The device can be uni-axially compressed or squeezed from a first configuration, such as a radially compacted configuration (e.g., as shown in Figure 1), into a second configuration, such as a radially expanded configuration (e.g., as shown in Figure 3). The uni-axial compression can be parallel to the longitudinal axis (in this application, longitudinal axis by itself refers to the longitudinal axis of the expandable support device as a whole). The expandable support device can get longitudinally shorter as the device radially expands.

(The terms expandable support device and stent are used interchangeably and non-limitingly throughout the remainder of the specification. In the claims, a stent is type of the expandable support device.)

The stent can transform the compressive force (i.e., from longitudinally applied work: longitudinally applied compressive force multiplied by a longitudinal distance that the stent is compressed) into a radial force (i.e., from the radially delivered work: radial expansion force multiplied by a radial distance that the stent is expanded). This force transformation can "gear up" the radial force from the longitudinal force. The stent can produce radial forces during expansion that can be from about 1 to about 50 times, yet more narrowly from about 10 times to about 30 times, the applied longitudinal compressive force.

The expandable support device can be configured to radially expand nonuniformly, uniformly in all angles from the longitudinal axis, along the length of the longitudinal axis, or combinations thereof.

The expandable support device can be configured to expand in multiple planes (i.e., multipanar expansion), in a single plane (i.e., uniplanar expansion), or combinations thereof. For example, during uniplanar expansion, the expandable support device can expand only taller (i.e., in a vertical plane), only wider (i.e., in a horizontal plane), or only in a plane not horizontal or vertical.

The expandable support device can have non-uniform radial expansion, for example the device can expand from a pre-deployed circular diameter of about 3 mm (0.1 in.) to a deployed rectangular configuration that can measure about 4 mm (0.1 in.) by about 6 mm (0.2 in.). The expandable support device can have radially contracted configurations with non-round cross sections, for example, square, triangular, rectangular, or combinations thereof. The expandable support device can have a tapered radially contracted and/or radially expanded configuration. The expandable support device can have open or closed ends that can allow or prevent fluid flow.

The porosity around the expandable support device can vary radially, angularly, and/or longitudinally (e.g., low to high, or even low in one section and high in other sections).

The expandable support device can be deployed to a treatment site fully or partly radially contracted or fully or partially radially expanded configuration.

The expandable support device can have a very high shear strength.

The expandable support device can be locked open by controlling the expandable support device length once the expandable support device is radially expanded. Locking or controlling the longitudinal length of the expandable support device length once the device is expanded can, for example, increase the maximum radial and shear forces sustainable by the expandable support device.

The expandable support device can be balloon expandable (e.g., deformable) or not balloon expandable or self-expandable (e.g., resilient).

The stent can be delivered using a uni-axial delivery/expansion system, such as those disclosed herein and in the references mentioned herein. The delivery system (i.e., deployment tool) can expand or contract. The deployment tool can have a sliding sheath shaft. The deployment tool can deploy the expandable support device uniformly from both longitudinal ends. Longitudinal compaction of the expandable support device can radially expand the expandable support device.

The expandable support device can be partially or completely radially expanded by rotating one end of the expandable support device relative to the other end of the expandable support device.

The expandable support device can have a small cross-sectional profile [0028] The expandable support device can have variable inner and/or outer diameters. The expandable support device can have a variable wall thickness (e.g., thick and thin spots radially, and/or angularly, and/or axially). The expandable support device can have variable cell dimensions, such as cell geometry (e.g., length, width, departure angle 72), gaps and offsets between cells, strut and/or link widths, uniformity of struts and cells, cells phase to one another. The first end of the expandable support device can have a different configuration that the second end of the expandable support device.

The expandable support device can have a locking trellis. The expandable support device can be made from a ductile metal.

### BRIEF DESCRIPTION OF THE DRAWINGS

Embodiments of apparatus in accordance with the present invention will now be described, by way of example only, with reference to the accompanying drawings. Not all of the subject-matter illustrated in the drawings is covered by the independent claim. In particular, Figs. 1-4, 10, 11, 26-28 and 36-55 show subject-matter that is covered by the independent claim.
Figure 1 illustrates an embodiment of the expandable support device in a first configuration.
Figure 2 illustrates a close up of a cell of the expandable support device of Figure 1.
Figure 3 illustrates an embodiment of the expandable support device in a second configuration.
Figure 4 illustrates a close up of a cell of the expandable support device of Figure 3.
Figure 5a is a close up view of an example of a portion of the expandable support device including two cells.
Figure 5b is a close up view of an example of a cell from Figure 5 a.
Figure 5c is a close up view of an example of a cell.
Figures 6 and 7 are close up views of various examples of a portion of the expandable support device including two cells.
Figures 8 and 9 illustrate an example of expanding the expandable support device with a deployment tool.
Figures 10 and 11 illustrate various embodiments of the expandable support device in an expanded configuration.
Figures 12 through 14 illustrate an example of expanding the expandable support device with a deployment tool.
Figure 15 illustrates an example of the expandable support device in a contracted configuration.
Figure 16 illustrates the expandable support device of Figure 15 in an expanded configuration.
Figure 17 illustrates an example of the expandable support device in a contracted configuration.
Figure 18 illustrates the expandable support device of Figure 17 in an expanded configuration.
Figures 19 through 21 illustrate various examples of the cell.
Figures 22 through 25 illustrate various examples of transverse cross- sections of the expandable support device in contracted (solid lines) and expanded (phantom lines) configurations
Figure 26 is a side view of an embodiment of the expandable support device in a contracted configuration.
Figure 27 is a side view of the expandable support device of Figure 26 in an expanded configuration.
Figure 28 is a perspective view of an embodiment of the expandable support device.
Figure 29 is a side view of an example of the expandable support device in a contracted configuration.
Figure 30 is a perspective view of the expandable support device of Figure 29. Figure 31 is a side view of the expandable support device of Figure 29 in an expanded configuration.
Figure 32 is a side view of an example of the expandable support device in a contracted configuration.
Figure 33 is a front view of the expandable support device of Figure 32.
Figure 34 is a side view of the expandable support device of Figure 29 in an expanded configuration.
Figure 35 is a front view of the expandable support device of Figure 29 in an expanded configuration.
Figure 36 is a side view of an embodiment of the expandable support device in a contracted configuration.
Figures 37 and 38 are side views of various embodiments of the expandable support device in an expanded configuration.
Figure 39 and 40 illustrate various embodiments of the expandable support device in an expanded configuration.
Figure 41 is a side view of an embodiment of the expandable support device in a contracted configuration.
Figure 42 is a front view of the expandable support device of Figure 41.
Figure 43 is a perspective view of the expandable support device of Figure 41.
Figure 44 is a side view of the expandable support device of Figure 41 in an expanded configuration.
Figure 45 is a front view of the expandable support device of Figure 44.
Figure 46 is a perspective view of the expandable support device of Figure 44.
Figure 47 illustrates an embodiment of the expandable support device.
Figure 48 is a perspective view of an embodiment of the expandable support device.
Figure 49 illustrates an embodiment of a flattened wall of the expandable support device of Figure 48.
Figure 50 is a perspective view of an embodiment of the expandable support device.
Figure 51 illustrates an embodiment of a flattened wall of the expandable support device of Figure 50.
Figure 52 is a perspective view of an embodiment of the expandable support device.
Figure 53 illustrates an embodiment of a flattened wall of the expandable support device of Figure 52.
Figure 54 is a perspective view of an embodiment of the expandable support device.
Figure 55 illustrates an embodiment of a flattened wall of the expandable support device of Figure 54.
Figure 56 is a perspective view of an example of the expandable support device.
Figure 57 illustrates an example of a flattened wall of the expandable support device of Figure 56.
Figure 58 is a sagittal cross-sectional view of an example of a method for deploying the expandable support device in a vertebra.
Figure 59 is a sagittal cross-sectional view of an example of a method for deploying the expandable support device in an intervertebral disc.
Figures 60 through 64 are transverse (i.e., horizontal) cross-sectional views of examples of various methods for deploying various examples of the deployed expandable support device.
Figures 65 and 66 illustrate an example of a method of using the expandable support device in the vasculature.
Figures 67 and 68 illustrate an example of a method of using the expandable support device in the vasculature.
Figure 69 is a perspective view of an example of a method of using the expandable support device across a valve, with the valve shown in cross-section.
Figure 70 illustrates an example of cross-section C-C of Figure 69.
Figure 71 is a perspective cross-section view of an example of a method of using the expandable support device across a valve.
Figure 72 illustrates an example of cross-section D-D of Figure 71.
Figure 73 illustrates an example of cross-section D-D of Figure 71.
Figure 74 is a perspective view of an example of a method of using the expandable support device across a valve, with the valve shown in cross-section.

### DETAILED DESCRIPTION

Figure 1 illustrates the expandable support device 14 in a contracted configuration. The expandable support device 14 can have a device first end 8 and a device second end 10. The device first end 8 and the device second end 10 can be at opposite longitudinal ends of the expandable support device 14. The expandable support device 14 can have an expandable support device wall 16. The expandable support device 14 can be configured as a cylinder. The expandable support device 14 can have one or more cells. The cells can be holes or voids in the expandable support device wall 16. The cells can be aligned in cell rows 4, cell columns 184, staggered, or randomly configured on the expandable support device 14.

A uni-axial compressive force, shown by arrows, can be applied on the device first end 8 and the device second end 10. The compressive force can produce a radial expansion 18, shown by arrows.

Figure 2 illustrates a single exemplary cell from the expandable support device 14. The cell can be formed by links 20 connected at joints 22 (e.g., hinges 24). The links 20 can be constrained, for example, to have no degrees of freedom within each link 20. The links 20 can be rigid and/or flexible. The joints 22 can be separate and discrete elements from the links 20, and/or sections of the expandable support device wall 16 that are designed to flex or bend when the compression force is applied. The cell can be a four-bar linkage. The cell can be in the closed configuration, as shown, before the compression force is applied.

Figure 3 illustrates the expandable support device 14 of Figure 1 in an expanded configuration. Figure 4 illustrates the cell of Figure 3 after radial expansion 18, as shown by arrows. The cell can be in an open configuration after radial expansion 18. The expandable support device 14 and/or cells can expand radially and contract longitudinally.

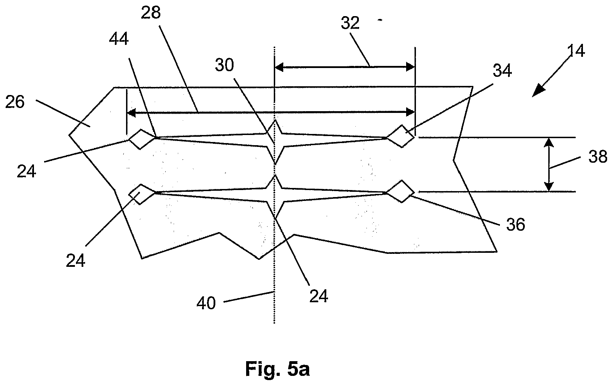

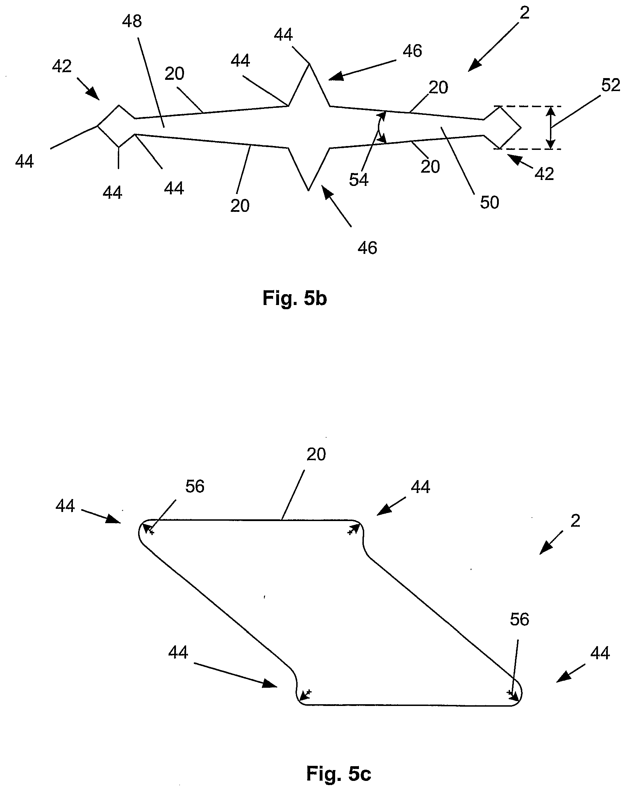

Figures 5a and 5b illustrate that the cells 2, such as a first cell 34, a second cell 36 (shown in Figure 5a), and other cells (not shown), can have two, three, four, or more hinges 24. The hinges 24 can have one or more degrees of rotational and/or translational freedom. The hinges 24 can have one or more hinge points 44. The device wall 26 can plastically deform and/or resiliently deform at the hinge points 44. The compression force applied along the longitudinal axis can cause the rotation at the hinge points 44. The hinge points 44 can have one, two, three, four, five, six or more degrees of rotational freedom. The hinge points 44 can have translational and/or rotational degrees of freedom. The device wall 26 can be made from any of the materials listed herein, for example a ductile metal or plastic, such as a polymer. The cells can rotate (i.e., flex and bend) similarly to a trellis or four-bar linkage, as shown supra.

The cell can have a cell length 28. The cell length 28 can be measured along the longitudinal axis of the expandable support device 14 and/or the cell longitudinal axis 30. The cell length 28 can be from about 0.1 mm (0.005 in.) to about 10 mm (0.5 in.), more narrowly from about 1.9 mm (0.075 in.) to about 8 mm (0.3 in.), for example about 5 mm (0.2 in.). The cell can have a branch length 32, for example from about 0.1 times the cell length 28 to about 0.9 times the cell length 28, more narrowly from about 0.25 times the cell length 28 to about 0.75 times the cell length 28, for example about 0.5 times the cell length 28 (i.e., the cell length 28 can be any cell length as disclosed herein). The transverse distance 40 along the expandable support device 14 between the first cell 34 and the second cell can be a cello height gap. The cell height gap 38 can be from about 0.1 mm (0.005 in.) to about 0.1 mm (0.1 in.), more narrowly from about 0.46 mm (0.018 in.) to about 1.5 mm (0.060 in.), for example about 0.76 mm (0.030 in.).

The cell can have a cell longitudinal axis 30. As shown, the first cell 34 can have a first cell longitudinal axis 66. The second cell can have a second cell longitudinal axis 68. The cell can have a first branch 48 and a second branch 50. Each branch can have a branch length 32. The branch length 32 can vary as the expandable support device 14 is deployed.

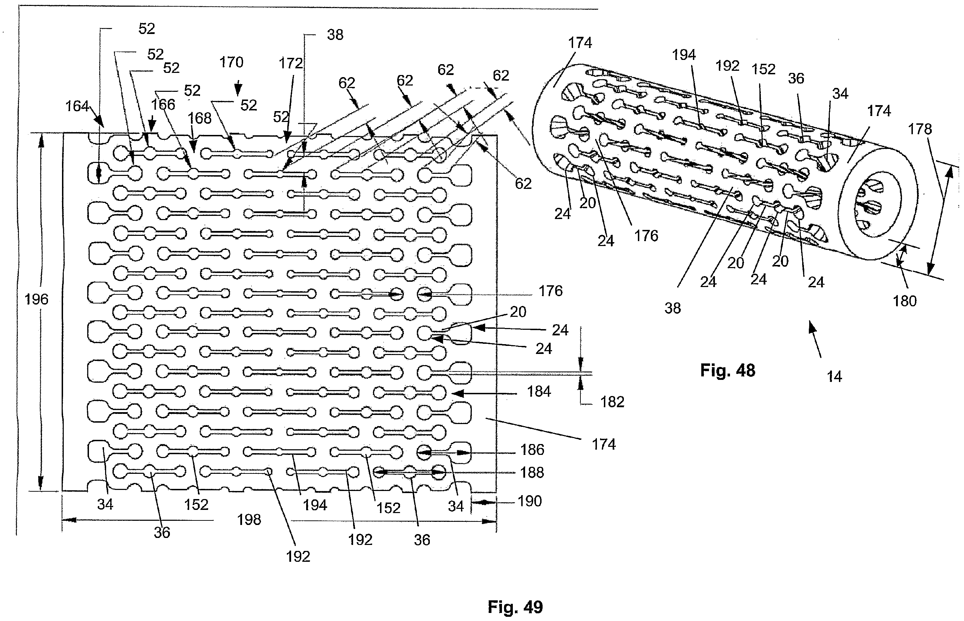

Figure 5b illustrates that the cell can have a first branch 48. The cell can have a second branch 50. One or more branches\can have two or more links 20. One or more branches can terminate in a hinge 24, for example a five-point hinge 42. The five-point hinge 42 can have five hinge points 44. The first branch 48 can attach to the second branch 50 at two hinges 24, for example three-point hinges 46. The hinges 24 can have hinge diameters 52. Examples for hinge diameters 52 within possible ranges of hinge diameters 52 are disclosed at least in Figure 49.

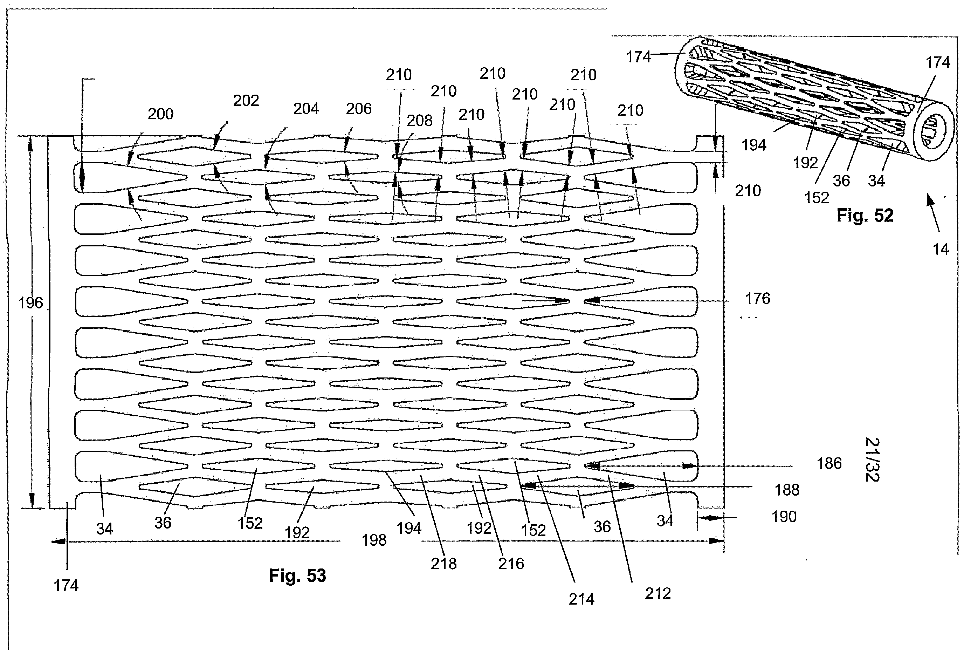

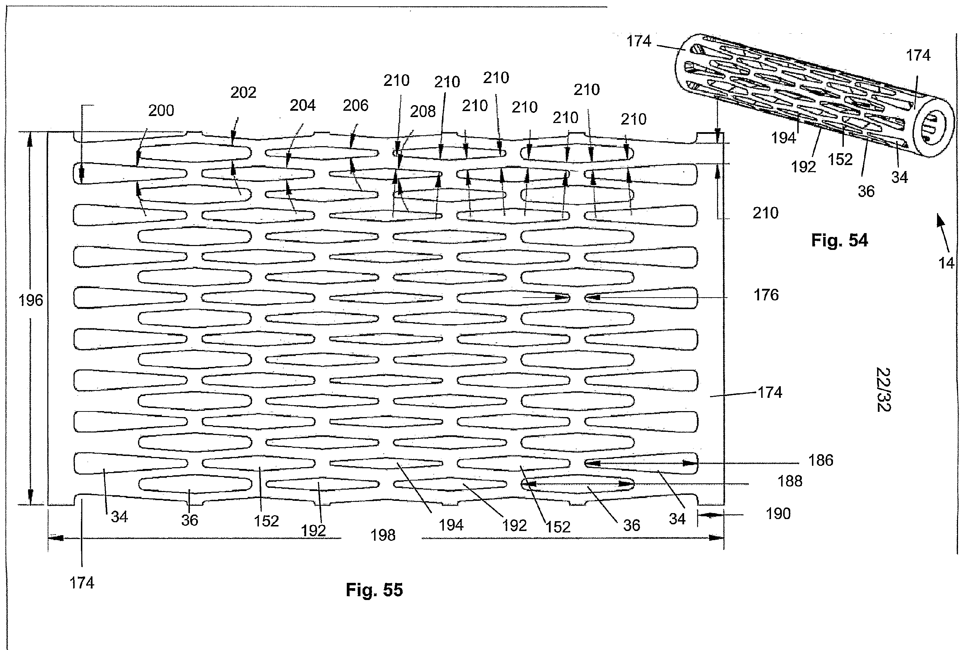

The angle between adjacent links 20 can be a link angle 54. Examples for link angles 54 within possible ranges of link angles 54 are disclosed at least in Figures 53 and 55. The hinges 24 can be configured to expand and/or contract the angle between the links 20 attached to the specific hinge 24.

Figure 5c illustrates that each hinge 24 can have hinge point radius 56. The hinge point radius 56 can be from about 0.1 mm (0.004 in.) to about 20 mm (0.8 in.), more narrowly from about 0.2 mm (0.008 in.) to about 4 mm (0.2 in.), for example about 1 mm (0.04 in.).

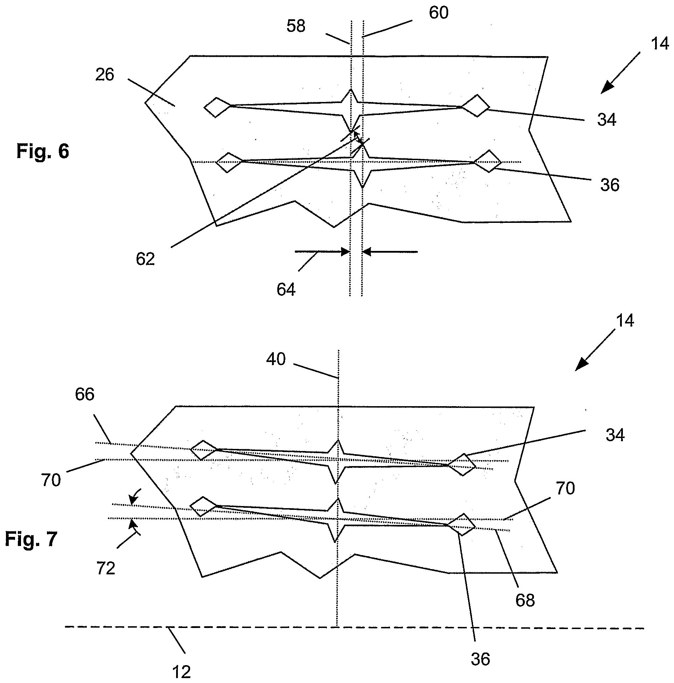

Figure 6 illustrates that the first cell 34 can have a first cell transverse axis 66. The second cell can have a second cell transverse axis 60. The transverse axis can intersect the center of area of the cell. The transverse axis can intersect the hinge points 44 between the links 20 on the first and second branches 50. The distance between the first cell transverse axis 66 and the second cell transverse axis 60 can be a cell row offset 64. The cell row offset 64 can be zero, as shown in Figure 5, or non-zero, as shown in Figure 6. The cell can have a hinge gap 62. The hinge gap 62 can be the distance from one hinge 24 to the closest hinge 24 on the adjacent cell.

Figure 7 illustrates that one or more cells can have a departure angle 72. The departure angle 72 can be the angle between the cell longitudinal axis and the longitudinal axis, or a longitudinal axis parallel 70. The departure angle 72 can be positive and/or negative from about 0 degrees to about 90 degrees, more narrowly from about 5 degrees to about 45 degrees, yet more narrowly from about 7.5 degrees to about 30 degrees, for example about 15 degrees.

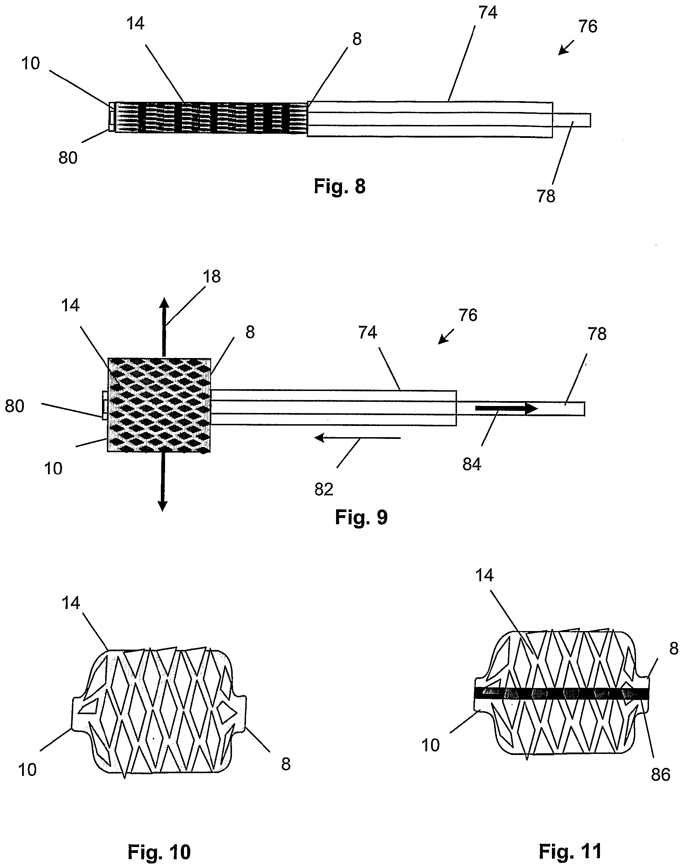

Figure 8 illustrates that the expandable support device 14, for example in a radially contracted configuration, can be loaded on a uni-axial deployment tool. The deployment tool can have a slide 78. The deployment tool can have a sheath or handle 74. The slide 78 can be slidably attached to the handle 74. The slide 78 and the handle 74 can have concurrent longitudinal axes (not explicitly shown). The slide 78 can be rotationally attached to the handle 74 with respect to the concurrent longitudinal axes. The slide 78 can be on the radial interior of the expandable support device 14. The slide 78 can be fixedly or rotationally attached to a tool butt or head. The tool head can releasably engage the device second end 10. The handle 74 can releasably engage the device first end 8.

Figure 9 illustrates that a translational force, as shown by arrow 84, can be applied to the slide 78 in a direction away from the expandable support device 14 while an opposite force is applied to the handle 74 resulting in a translation, as shown by arrow 82, of the slide 78 with respect the handle 74. The translation shown can occur during deployment of the expandable support device 14. The tool head and the handle 74 can longitudinally compress the expandable support device 14. The expandable support device 14 can radially expand, as shown by arrows. The expandable support device 14 can longitudinally shorten.

Figure 10 illustrates that when the expandable support device 14 is in the expanded configuration, the device first end 8 and/or the device second end 10 can have a radius equivalent to the radius of the device first end 8 and/or the device second end 10 in the contracted configuration. The remainder (i.e., other than the device first end 8 and/or the device second end 10) of the expandable support device 14 can expand radially outward. The first and/or second device ends of the configuration of the expandable support device 14 shown in Figure 10 can be constrained (i.e., attached) to the deployment tool, such as the deployment tool shown in Figures 8 and 9, during deployment.

Figure 11 illustrates that the expandable support device 14 can have a locking bar 86. The locking bar 86 can be fixedly or releasably attached to the first device end and/or the second device end before and/or during and/or after deployment of the expandable support device 14. The locking bar 86 can be the length of the expandable support device 14 in the radially expanded configuration, as shown. The locking tension bar can increase radial and shear forces.

The first device end and/or the second device ends can be completely and/or substantially closed. The closed device ends can create a hollow cavity in the device. The hollow cavity, with or without closed ends, can be filled with any material disclosed herein, for example, bone, bone chips, cement, bone morphogenic protein/powder (BMP), drugs, ceramics, small balls of any of the above, or combinations of the above.

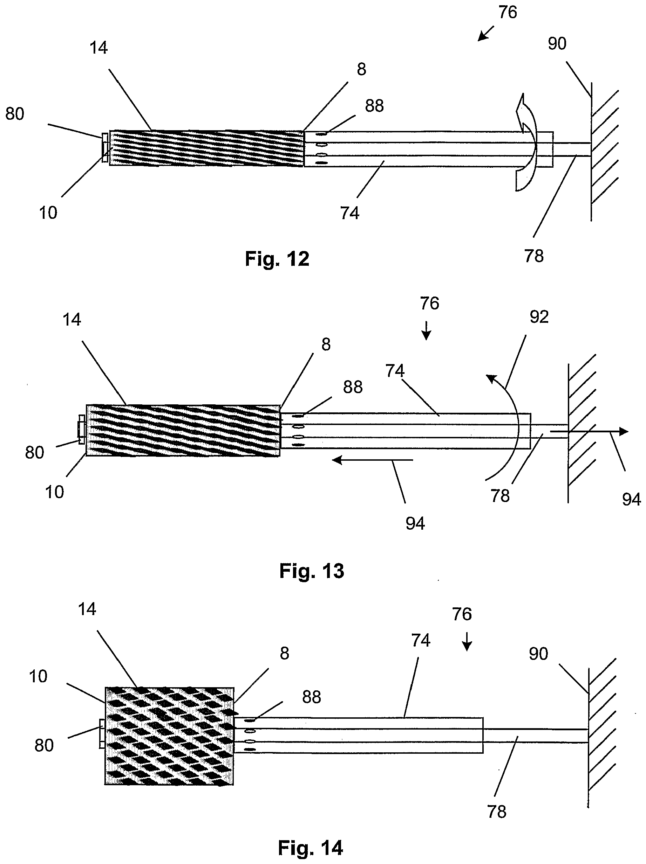

Figures 12 through 14 illustrate a method of deploying the expandable support device 14. The deployment tool can transmit a rotation force to the expandable support device 14. The slide 78 can be rotationally fixed to a fixator 90. The fixator 90 can be a relatively rotationally stationary element of the deployment tool, or a relatively rotationally stationary separate element (e.g., a surgeon's hand or a wall). The deployment tool can have a cam system to twist and/or allow twisting of the slide 78 with respect to the handle 74, for example from about 5 degrees to about 20 degrees.

The deployment tool can be hollow, for example allowing fluid to flow through the deployment tool. The slide 78 and/or handle 74 can be hollow. The slide 78 and/or handle 74 can have tool ports 88. The tool ports 88 can allow flow into and through the deployment tool 76 (e.g., the handle 74 and/or slide 78). The device second end 10 and/or the slide cap 80 can have a port in fluid communication with the hollow of deployment tool.

As shown in Figure 12, the handle 74 can be rotated, as shown by the arrow, with respect to the slide 78. The handle 74 can be removably attached (e.g., rotationally or rotationally and translationaly fixed) to the expandable device at the device first end 8. The slide 78 can be removably attached (e.g., rotationally or rotationally and translationaly fixed) to the expandable device at the device second end 10, for example at a slide cap 80.

As shown in Figure 13, the handle 74 can be translated, as shown by arrows 94, with respect to the slide 78 after and/or during rotation 92 of the handle 74 with respect to the slide 78 (e.g., rotation to unlock - for example with audible and/or tactile feedback such as a click - the handle 74 from the slide 78 and translation to compress the expandable support device 14, and/or partial rotation to enable easier translational longitudinal compression 162 and radial expansion 18 of the expandable support device 14 followed by the translational longitudinal compression 162 and radial expansion 18).

Figure 14 illustrates that the handle 74 can be further rotated and/or translated with respect to the slide 78 to fully expand the expandable support device 14. The expandable support device 14 can expand similar to the untwisting of a coil spring. This "deployment twist" can be used to open the cells partially and/or completely. If the device is twisted slightly, the uni-axial compression method disclosed herein can then be used to complete deployment/expansion of the device.

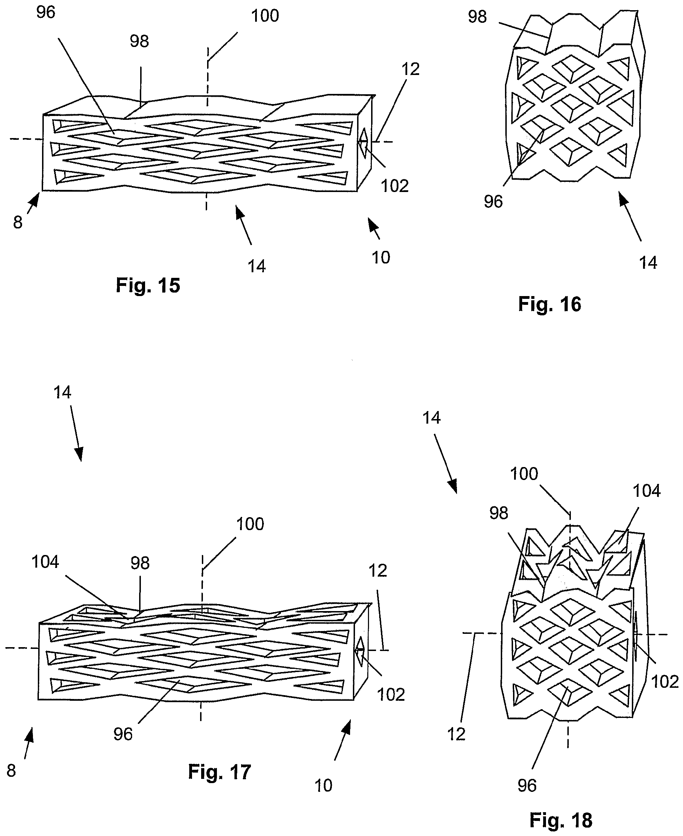

Figure 15 illustrates that the expandable support device 14 can have an expandable device transverse axis 100 at a right angle to the expandable device longitudinal axis 12. The expandable support device 14 can have horizontal cells 96 on one side or two opposing sides of the expandable support device 14. The compression folds 98 can be designed to encourage compression at the fold. The horizontal cells 96 can be partially and/or fully diamond-shaped. Figure 15 shows the expandable support device 14 in a radially compressed and longitudinally expanded configuration.

The expandable support device 14 can have an end cell 102 at the device first end 8 and/or the device second end 10. The end cells 102 can be configured to engage the deployment tool (not shown). The end cells 102 can be configured to engage the locking bar 86. The end cell 102 at the device first end 8 can be a different geometry and size than the end cell 102 at the device second end 10. The expandable support device 14 can have one or more compression folds 98.

The expandable support device 14 can have a round (e.g., circular, oval), tapered, triangular or square longitudinal cross-section. The expandable support device 14 with the square longitudinal cross-section can be used the same as the expandable support device 14 with the round cross section. The expandable support device 14 with the square longitudinal cross-section when in a radially compressed configuration can have a substantially square and/or round longitudinal cross-section when in a radially expanded configuration. The expandable support device 14 with the round longitudinal cross-section when in a radially compressed configuration can have a substantially square and/or round longitudinal cross-section when in a radially expanded configuration. The expandable support device 14 with the square longitudinal cross-section when in a radially compressed configuration can have a substantially different longitudinal cross-section when in a radially expanded configuration than the longitudinal cross-section in a radially expanded configuration of the expandable support device 14 with the round longitudinal cross-section when in a radially compressed configuration.

Figure 16 illustrates that expandable support device 14 of Figure 15 in a radially expanded and longitudinally compressed configuration. The expandable support device 14 can be configured to have no cells on the top and/or bottom surface.

Figure 17 illustrates an expandable support device 14 similar to the expandable support device 14 of Figure 15, but with vertical cells 104 in the top and/or bottom of the expandable support device 14. Figure 18 illustrates the expandable support device 14 of Figure 17 in a radially expanded and longitudinally compressed configuration. The vertical cells 104 and/or the horizontal cells 96 can be closed and/or open in the radially expanded configuration.

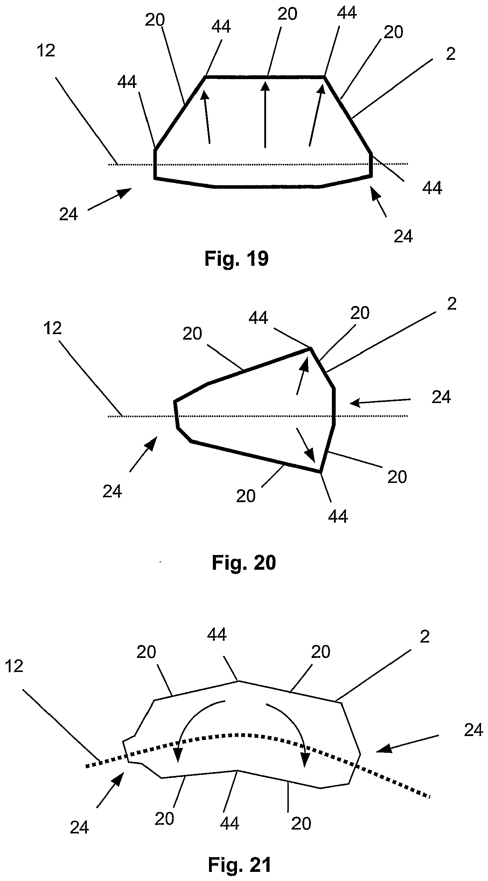

Shape and density changes of the expandable support device 14 during radial expansion 18 can be altered by different designs of the cell geometry. Figure 19 illustrates the cell in a radially expanded configuration. The cell can be configured to expand in a single plane, as shown by arrows. The cell can be configured to expand in a single or substantially singular direction (e.g., translating and not rotating one or more links 20), as shown by arrows. The hinge points 44 and links 20 can be configured as shown to allow for uniplanar expansion.

The expandable device longitudinal axes shown in Figures 19 through 21 can be of configurations after the expandable support devices 14 are radially expanded.

Figure 20 illustrates the cell in a radially expanded configuration. The cell can be configured to allow expansion in a tapered configuration, as shown by arrows. Expansion in a tapered expansion can include translational expansion of two opposite hinge points 44 near the same longitudinal or transverse end of the cell.

Figure 21 illustrates the cell in a radially expanded configuration. The cell can be configured to allow expansion in a curved configuration, as shown by arrows. Expansion in a curved expansion can include rotation in the same direction by two or more substantially or completely opposite hinge points 44 on opposite sides of the cell.

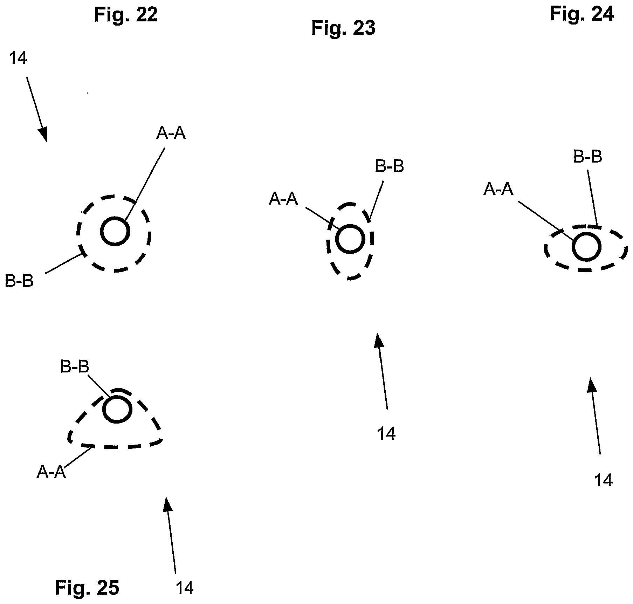

Figures 22 through 25 illustrates that the expandable support device 14 can have a radially contracted configuration with a substantially circular cross-section A-A. The expandable support device 14 can be configured, for example due to cell configuration, to radially expand to a cross-section B-B that can be circular, as shown in Figure 22. The expandable support device 14 can be configured, for example due to cell configuration, to radially expand to a cross-section B-B that can be oval having a major axis in a first direction, as shown in Figure 23. The expandable support device 14 can be configured, for example due to cell configuration, to radially expand to a cross-section B-B that can be oval having a major axis in a second direction (e.g., at a right angle to the first direction), as shown in Figure 24. The expandable support device 14 can be configured, for example due to cell configuration, to radially expand to a cross-section B-B that can be substantially or completely triangular (e.g., with and/or without rounded corners), as shown in Figure 25.

The expandable support device 14 with a circular cross-section A-A in a radially non-expanded configuration can radially expand to a non-circular cross-section B-B, for example, due to varying cell geometries (e.g., radial and/or angular/transverse and/or longitudinal variations of each cell and/or from cell to cell). The radially-expanded cross-section B-B can be centered or not centered on the radially non-expanded cross-section A-A, for example, due to varying cell geometries (e.g., radial and/or angular/transverse and/or longitudinal variations of each cell and/or from cell to cell).

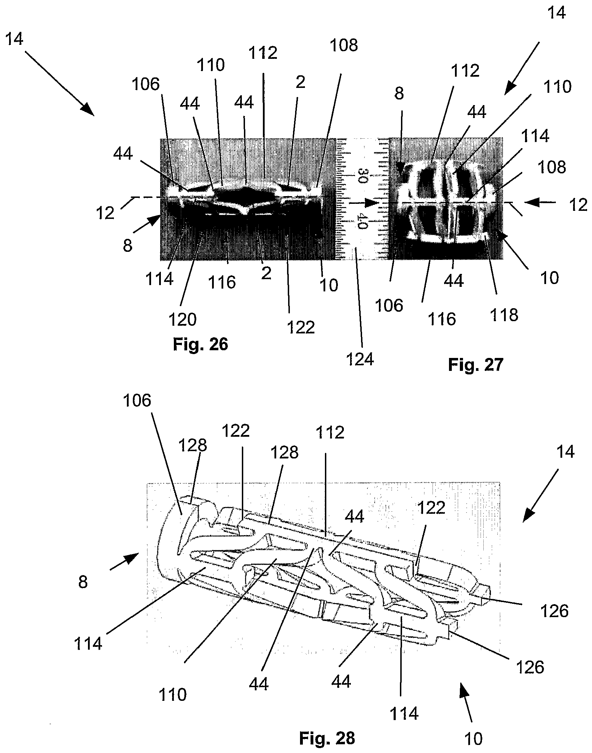

Figures 26 and 27 illustrate the expandable support device 14 in a radially contracted configuration and a radially expanded configuration, respectively. The expandable support device 14 can be compressed along the expandable device longitudinal axis 12 to radially expand. The links 20 can rotate at the hinge points 44 during radial expansion 18 and contraction.

The expandable support device 14 has a first end ring 106 at the device first end 8. The expandable support device 14 can have a second end ring 108 at a device second end 10. One or both end rings 174 can engage the deployment tool (not shown) during deployment including longitudinal compression 162.

The expandable support device 14 can have side links 20 extending from the end rings 174, for example each at a fixed (as shown) or hinge point 44. Cross links 110 can extend from the side links 114, for example each at a fixed or hinge (as shown) point 44. Top 112 and bottom links 116 can extend from the cross links 110, for example each at a hinge (as shown) point 44. The top, bottom and side links 114 can be substantially parallel to the expandable device longitudinal axis 12. The cross links 110 can be substantially not parallel to the expandable device longitudinal axis 12. One having ordinary skill in the art understands that the orientation of the expandable support device 14 can turn side links 114 into top 112 or bottom links 116 and vice versa, as well as top links 112 into bottom links 116 and vice versa.

The bottom 116 and top 112 links can terminate in first end proximal tips 120 and second end proximal tips 122. Each side of the expendable support device can have one, two or more side links 114, for example in (as shown) and/or out of line with each other and overlapping and/or not overlapping (as shown).

Figure 27 illustrates the longitudinal compressive force 6, shown by arrows, that can be applied to the end rings 174, for example to radially expand the expandable support device 14. A longitudinal tensile force can be applied at the end rings 174, for example, to radially contract the expandable support device 14. The longitudinal forces can be applied aligned with the longitudinal axis (i.e., the center as seen in cross-section A-A or B-B) of the expandable support device 14.

Figure 28 illustrates that the expandable support device 14 can have one, two or more second end distal tips 126 and/or second end proximal tips 122 at the second end. Either device end can have no end ring 174. The distal and/or proximal tips 118 can engage the deployment tool (not shown) during deployment including longitudinal compression 162.

The expandable support device 14 can have a seam 128. The seam 128 can partially or completely internally separate individual links 20 and/or the end rings 174. For example, the seam 128 can completely internally separate the first end ring 106 and the top link 112, as shown. The top link 112 and/or the first end ring 106 can slide 78 against itself at the seam 128, for example, encouraging radially expansion in the direction of the seam 128 during longitudinal compression 162.

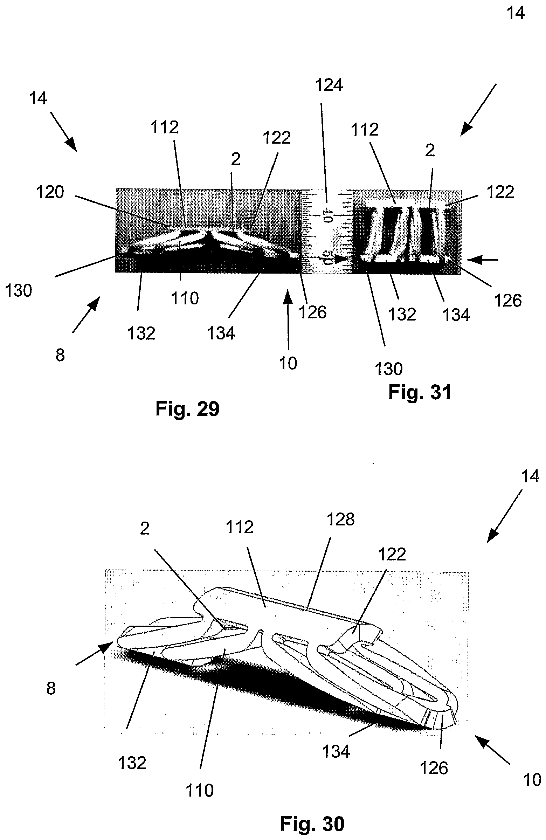

Figures 29 and 30 illustrate that the expandable support device 14 can have a top link, cross links 110, and one, two (as shown), or more bottom links 116. A first bottom link 132 and a second bottom link 134 can be in (as shown) and/or out of line with each other and overlapping and/or not overlapping (as shown).

The expandable support device 14 can have no end rings 174. The first bottom link 132 can have a first end distal tip 130. The top link 112 can have a first end proximal tip 120. The second bottom link 134 can have a second end distal tip 126. The top link 112 can have a second end proximal tip 122.

Figure 31 illustrates that the longitudinally compressive force 6, as shown by arrows, can be applied at the first end distal tip 130 and second end distal tip 126, for example to cause radial expansion 18 of the expandable support device 14. The longitudinal tensile force can be applied at the first end distal tip 130 and the second end distal tip 126, for example, to radially contract the expandable support device 14. The longitudinal forces can be applied unaligned with the longitudinal axis of the expandable support device 14.

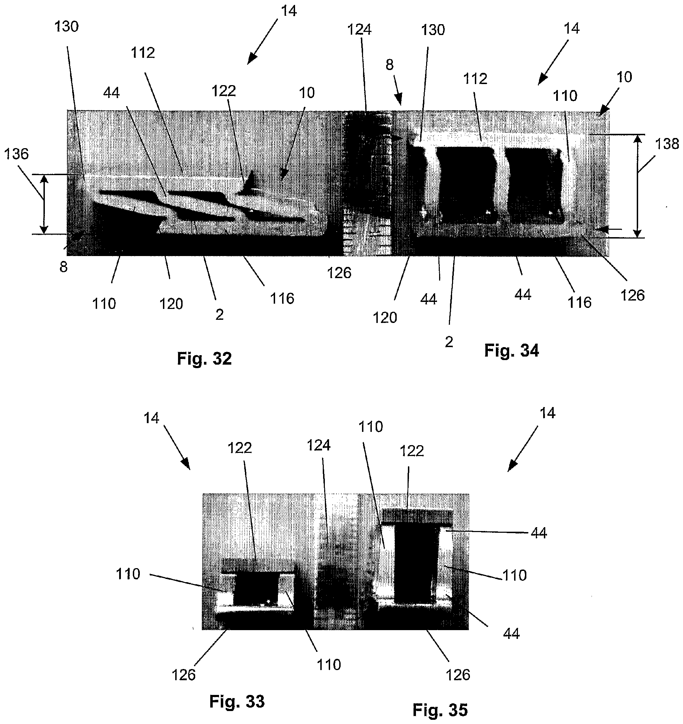

Figures 32 and 33 illustrates that the top link 112 and/or the bottom link 116 can be flat. The cross links 110 can extend from the top link 112 and/or bottom link 116 at an about 90° angle from the outer surface of the top link 112 and/or bottom link 116.

Figures 34 and 35 illustrate that the longitudinally compressive force 6, as shown by arrows, can be applied at the first end distal tip 130 and second end distal tip 126, for example to cause radial expansion 18 of the expandable support device 14. A longitudinal tensile force can be applied at the first end distal tip 130 and the second end distal tip 126, for example, to radially contract the expandable support device 14. The longitudinal forces can be applied on substantially diametrically opposite corners of the expandable support device 14.

The expandable support device 14 can have a in a radially contracted and/or radially expanded configuration can have a square or rectangular cross-section A-A and/or B-B. The expandable support device 14 can have a radially contracted height 136 and a radially expanded height 138. The radially contracted height 136 can be from about 2 mm (0.08 in.) to about 50 mm (2.0 in.), more narrowly from about 5 mm (0.2 in.) to about 20 mm (0.79 in.), yet more narrowly from about 6 mm (0.2 in.) to about 12 mm (0.47 in.), for example about 8.6 mm (0.33 in.), also for example about 8.0 mm (0.3 in.). The radially expanded height 138 can be from about 3 mm (0.1 in.) to about 100 mm (3.94 in.), more narrowly from about 10 mm (0.39 in.) to about 40 mm (1.6 in.), yet more narrowly from about 12 mm (0.47 in.) to about 24 mm (0.94 in.), for example about 15.6 mm (0.614 in.), also for example about 16.0 mm (0.630 in.), also for example about 18.1 mm (0.712 in.).

As shown in Figure 34, the expandable support device 14 in a radially expanded configuration can withstand post-deployment vertical compressive forces 6 (i.e., coming from the top and bottom of the figures) of, for example about 2.02 kN (455 lbs.), also for example about 3.11 kN (700 lbs.), also for example about 4.00 kN (900 lbs.), also for example more than about 4.38 kN (985 lbs.) without collapse or other failure. After radial compression loading, the radially expanded height 138 can reduce from the original radially expanded height 138 about 0.1% to about 20%, more narrowly from about 0.3% to about 15%, for example about 11%, also for example about 4.4%, also for example about 0.6%.

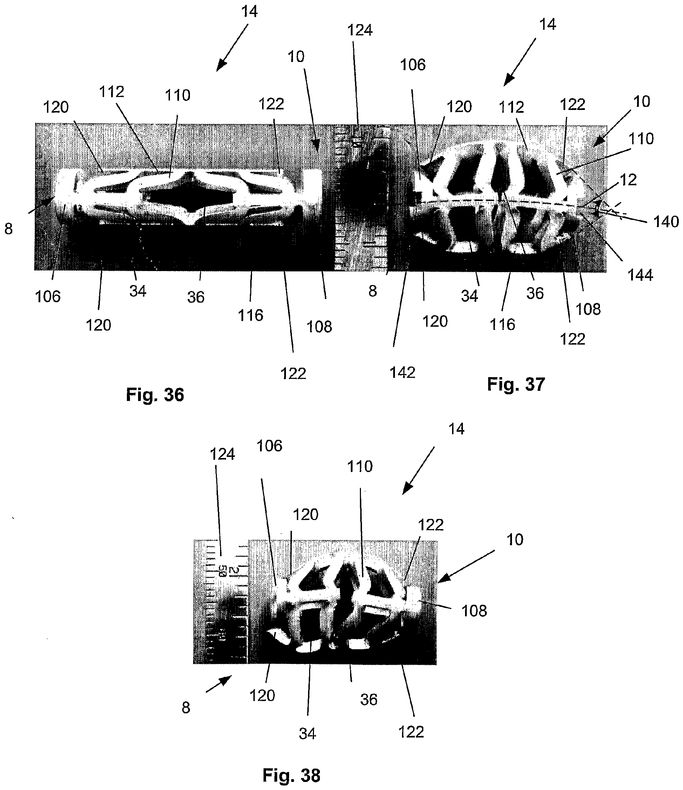

Figure 36 illustrates an expandable support device 14 similar to the embodiment shown in Figure 26. Figure 37 illustrates that the first end ring 106 can have a first engagement notch 142. The second end ring 108 can have a second engagement notch 144. The engagement notches 158 can be pre-formed and/or formed during use by the deployment tool 76. The engagement notches 158 can be lined and/or coated with a hardened material.

Any combination or all of the tips (e.g., first end proximal tips 120 and second end proximal tips 122) and/or the top and/or bottom and/or side links 114 can bend toward the expandable device longitudinal axis 12, for example so lines extending (as shown) from the tips would substantially intersect the expandable device longitudinal axis 12 at a taper angle 140. The taper angle 140 can be from about 20° to about 70°, for example at about 45° (as shown). The expandable device longitudinal axis 12 can bend during radial expansion 18.

Figure 38 illustrates that the longitudinal compression 162 force can be large enough to compress the end rings 174 toward the center of the expandable support device 14 past one or more tips. For example, the first end ring 106 can be compressed toward the center of the expandable support device 14 past one of the first end proximal tips 120, as shown. The tips can have barbs, hooks, pins, anchors, or combinations thereof.

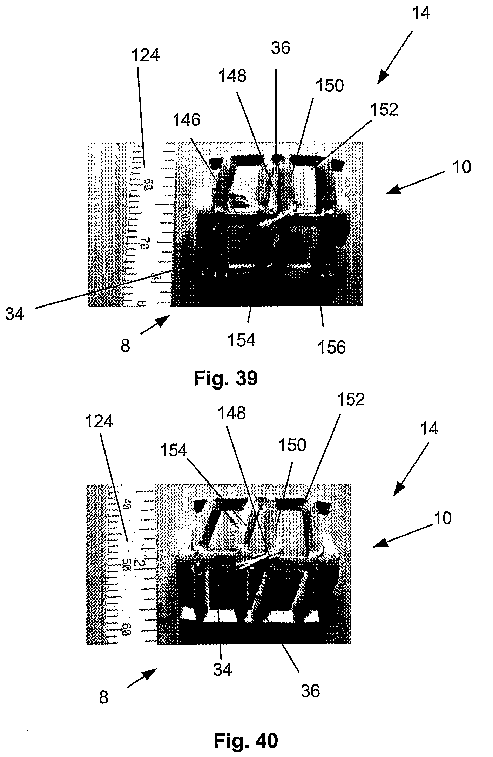

Figures 39 and 40 illustrate that one or more wires 148, filaments, fibers or combinations thereof can be threaded through adjacent or nearby cells. The expandable support device 14 can have a first side link 146 and a first cross link 154 partially defining the first cell 34. The expandable support device 14 can have a second side link 156 and/or a second cross link 150 partially defining a third cell 152. The first cell 34 can be diametrically opposed to the third cell 152, for example, when the expandable support device 14 is in a radially expanded configuration. The wires 148 can be wrapped between the first cell 34 and the third cell 152, as shown. The wires 148 can be wrapped from about one to about 10 turns, for example two turns, as shown in Figures 39 and 40. The wires 148 can have a diameter, for example from about (0.003 in.) to about (0.100 in.), for example about (0.020 in.). The wire 148 can be any material listed herein, for example a metal (e.g., stainless steel, Nitinol, Elgiloy), and/or a polymer (e.g., PTFE, PET, PE, PLLA).

Figure 40 illustrates that the expandable support device 14 can be radially compressed, for example the expanded height 138 can reduce from about 1% to about 10%, more narrowly from about 3% to about 8%, for example about 5%.

The rulers 124 shown in Figures 26, 27, 29, 31, are numbered every 10 mm, and marked to the 1 mm on one side and 0.5 mm on the opposite side. The rulers 124 shown in Figures 32-40 are numbered every 10 mm and every 1 in., and marked to the 1 mm and the 1/16 in.

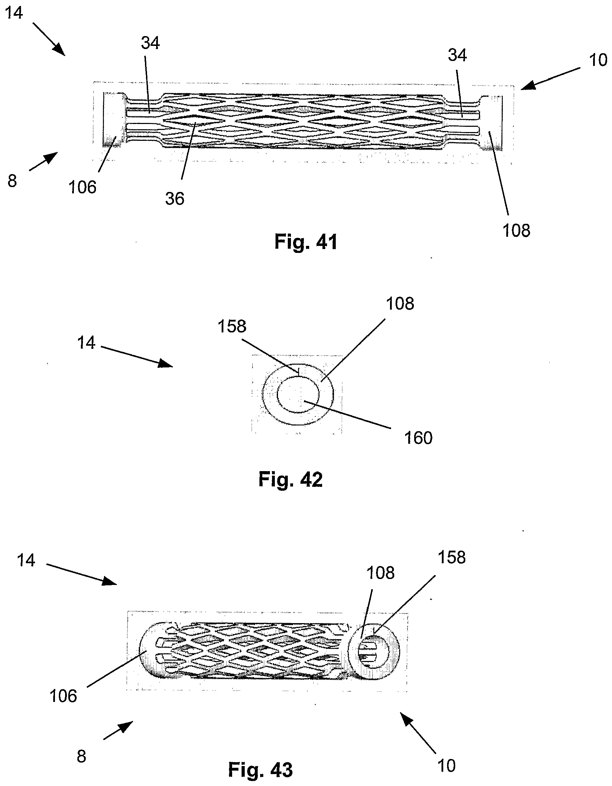

Figures 41 through 43 illustrate the expandable support device 14 that can be in a radially contracted configuration. In the radially contracted configuration, the expandable support device 14 can have longitudinally elongated first cells 34, for example, at the device first end 8 and/or the device second end 10. In the radially contracted configuration the first cells 34 can partially or completely have a smaller outer diameter 196 than the remainder of the expandable support device 14. In the radially contracted configuration, the expandable support device 14 can have one or more cell rows 4 of diamond-shaped second cells, for example, between one or two cell rows 4 of the first cells 34. The expandable support device 14 can have one or more engagement notches 158 on the first 106 and/or second end rings 108.

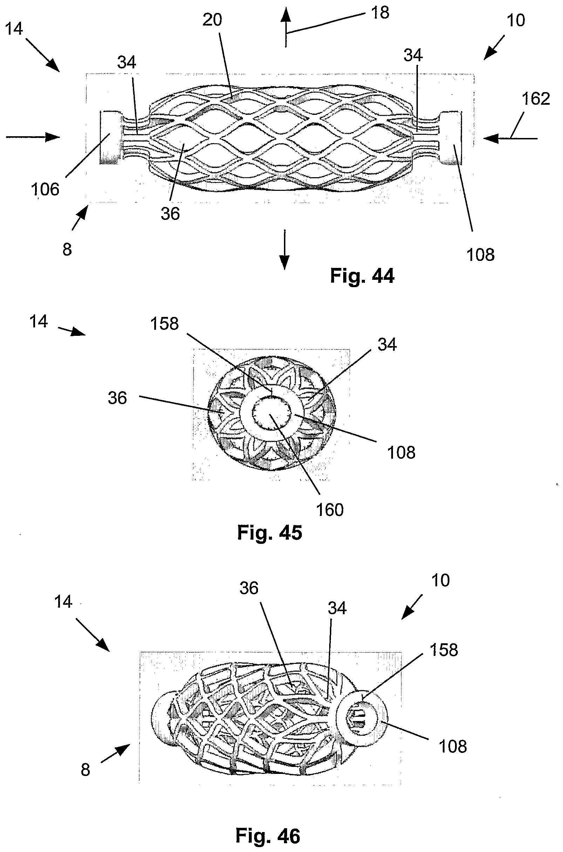

Figures 44 through 46 illustrate that as the expandable support device 14 is longitudinally compressed, the expandable support device 14 can radially expand, as shown by arrows. The expandable support device 14 at the cell rows 4 of the first cells 34 can radially contract, stay radially constant, or radially expand to a lesser degree than the second cell rows 4, during longitudinal compression 162 of the expandable support device 14. The first cells 34 can be five-link or three-link cells. One or more links 20 in a cell can be formed by the end ring 174. The second cells can be four-link cells.

The expandable support device 14 can have a patent internal channel 160. The internal channel 160 can extend from the device first end 8 to the device second end 10. The internal channel 160 can allow fluid flow 296 through the expandable support device 14 when in a radially contracted and/or radially expanded configuration. When in a radially expanded and/or radially contracted configuration, fluid can flow through the cells.

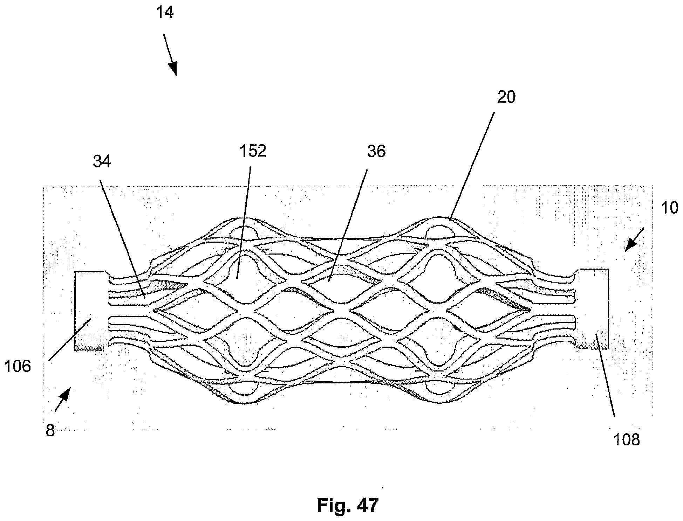

Figure 47 illustrates that the expandable support device 14 can have first cells 34, second cells and third cells. The third cells can radially protrude in part or whole from the remainder of the expandable support device 14. The configuration of the cells can vary with respect to the expandable device longitudinal axis 12.

All dimensions shown in Figures 49, 51, 53, and 55 can be exact or substantially approximate. All dimensions shown in Figures 49, 51, 53, and 55 can be examples within possible ranges.

Figure 48 illustrates that the expandable support device 14 can have first 34, second 36, third 152, fourth 192 and fifth 194 cells. The first 34, second 36, third 152, fourth 192 and fifth 194 cells can be in one or more first 164, second 166, third 168, fourth 170 and fifth 172 cell rows, respectively. The cell rows 4 can overlap each other. The cells in a single cell row 4 can progressively increase and/or decrease in one or more dimensions. The cells from one cell row 4 to the next can progressively increase and/or decrease in one or more dimensions. The cells can be arranged in one or more, for example eight, cell columns 184. The cells in a single cell column 184 can progressively increase and/or decrease in one or more dimensions. The cells from one cell column 184 to the next cell column 184 can progressively increase and/or decrease in one or more dimensions. The number of cells can vary from one cell column 184 to the next.

Figure 49 illustrates a flattened or unrolled wall of an expandable support device 14, or a cylindrical projection of the expandable support device 14. The expandable support device 14 can have an outer circumference 196 and a device length 198.

The outer circumference 196 can be from about 24.9 mm (0.981 in.) to about 25.2 mm (0.992 in.), more narrowly from about 24.9 mm (0.981 in.) to about 25.1 mm (0.987 in.) or from about 25.0 mm (0.986 in.) to about 25.2 mm (0.992 in.), for example about 25.0 (0.984 in.) or for example about 25.1 mm (0.989 in.).

The device length 198 can be from about 27.1 mm (1.067 in.) to about 45.8 mm (1.803 in.), more narrowly from about 27.1 mm (1.067 in.) to about 27.3 mm (1.073 in.) or from about 30.4 mm (1.197 in.) to about 45.8 mm (1.803 in.), yet more narrowly from about 30.4 mm (1.197 in.) to about 30.6 mm (1.203 in.) or from about 45.6 mm (1.797 in.) to about 45.8 mm (1.803 in.), for example about 27.4 mm (1.080 in.) or about 30.5 mm (1.200 in.), or about 45.7 mm (1.800 in.).

(The dimensions listed below for Figures 49 through 57 are representative for the expandable support device 14 in a radially contracted configuration and have tolerances of ±0.08 mm (±0.003 in.). Further none of the dimensions listed herein are limiting, and additional exemplary dimensions are shown in those figures having length scales.)

The expandable support device 14 can have one or more of the cells. The cells can be arranged orthogonally in cell columns 184 and cell rows 4. The first 164, second 166, third 168, fourth 170 and fifth 172 cell rows can have the first 34, second 36, third 152, fourth 192 and fifth 194 cells, respectively. The cells in a first cell column 184 can be aligned or unaligned (e.g., staggered) with the cells in an adjacent cell column 184. The cells can be symmetric about a longitudinal center of the expandable support device 14.

The cells in a single cell column 184 can be separated by cell row gaps 176. The cell row gap 176 can be about 1.01 mm (0.040 in.). The nearest distance between links 20 of cells in adjacent cell columns 184 is a cell height gap 38. The cell height gap 38 can be from about 0.559 mm (0.022 in.) to about 1.70 mm (0.067 in.), for example about 0.559 mm (0.022 in.) or about 1.1 mm (0.045 in.) or about 1.70 mm (0.067 in.).

The first cell 34 can have a first cell width 186. The second cell can have a second cell width 188. The first cell width 186 can be from about 3.81 mm (0.150 in.) to about 8.71 mm (0.343 in.), for example about 3.81 mm (0.150 in.) or about 7.62 mm (0.300 in.) or about 8.71 mm (0.343 in.). The second cell width 188 can be from about 5.08 mm (0.200 in.) to about 8.71 mm (0.343 in.), for example about 5.08 mm (0.200 in.) or about 7.62 mm (0.300 in.) or about 8.71 mm (0.343 in.). The third 152, fourth 192 and fifth 194 cells can have cell widths 226 equivalent to the first 186 and/or second cell widths 188.

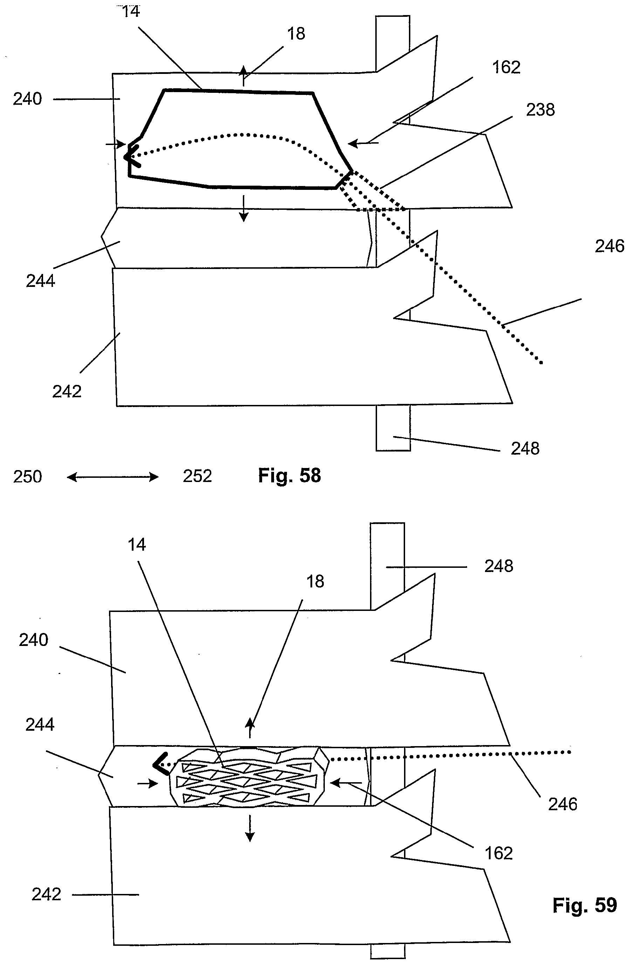

Each cell can have two, three or more hinges 24. The hinges 24 on a single cell can be connected to the other hinges 24 by links 20, as shown. When the expandable support device 14 is in a radially contracted configuration, as shown in Figures 59 through 57, The hinges 24 can be radially enlarged portions of the cell. The hinges 24 can be configured so that during longitudinal compression 162 of the expandable support device 14, the hinges 24 rotate to transfer the compressive energy to a radial energy, thereby radially expanding the expandable support device 14.

The hinges 24 can have hinge diameters 52 from about 0.51 mm (0.020 in.) to about 1.5 mm (0.059 in.), for example about 0.51 mm (0.020 in.) or about 1.0 mm (0.040 in.) or about 0.89 mm (0.035 in.) or about 0.76 mm (0.030 in.) or about 0.64 mm (0.025 in.) or about 0.51 mm (0.020 in.).

The hinges 24 can be a hinge gap 62 distance to the nearest hinge 24 on the adjacent cell column 184 cell. The hinge gaps 62 can be from about 0.71 mm (0.028 in.) to about 1.1 mm (0.042 in.).

The links 20 can have link heights 182 (i.e., slot gaps). The link heights 182 can be from about 0.25 mm (0.010 in.) to about 1.7 mm (0.067 in.), for example about 0.25 mm (0.010 in.) or about 1.7 mm (0.067 in.).

The expandable support device 14 can have a device length 198, a wall thickness 180 and an outer diameter 196. The device length 198 can be from about 27.18 mm (1.070 in.) to about 45.72 mm (1.800 in.), for example about 27.18 mm (1.070 in.) or 30.48 mm (1.200 in) or about 45.72 mm (1.800 in.). The wall thickness 180 can be from about 1.2 mm (0.049 in.) to about 1.7 mm (0.065 in.), for example about 1.2 mm (0.049 in.) or about 1.7 mm (0.065 in.). The outer diameter 196 can be from about 6.35 mm (0.250 in.) to about 10.2 mm (0.400 in.), for example about 7.95 mm (0.313 in.) or about 7.98 mm (0.314 in.).

The expandable support device 14 can have end rings 174 at one or both longitudinal ends of the expandable support device 14. The end rings 174 can have an end ring width 190. The end ring width 190 can be about 1.8 mm (0.070 in.).

The cells in the interior of the expandable support device 14 can have larger and/or smaller cell widths 226, hinge gaps 62, hinge diameters 52, link heights 182, cell row gaps 176, than cells near the ends of the expandable support device 14. Any or all of the dimensions of the elements, configurations, features, and characteristics of the expandable support device 14 can be symmetric about the longitudinal center (i.e., center radial plane) of the expandable support device 14, as shown.

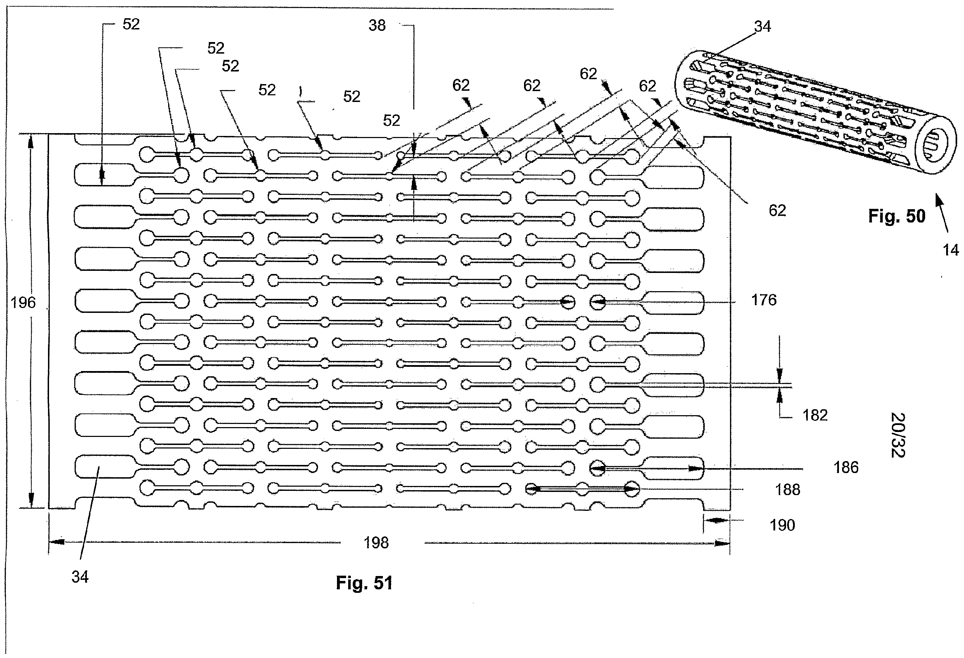

Figure 50 and 51 illustrate an embodiment of the expandable support device 14 similar to the expandable support device 14 shown in Figures 48 and 49, but with different exemplary dimensions.

Figures 52 and 53 illustrate an expandable support device 14 that can have diamond-shaped (e.g., four-strut or four-link 20) cells. The expandable support device 14 can have three-link (e.g., three-strut) or five-link (e.g., five-strut) cells, for example at the cell row 4 at each end of the expandable support device 14. The expandable support device 14 can have, respectively from the longitudinal end to the longitudinal middle of the expandable support device 14, first struts 212, second struts 214, third struts 216 and fourth struts 218. The struts nearer the longitudinal middle of the expandable support device can have a greater or lesser strut thickness 210. Each strut can have a variable or constant strut thickness over the length of the strut. The end of each strut near the longitudinal end of the expandable support device 14 can be thicker or thinner than the end of the same strut nearer the longitudinal middle of the expandable support device 14.

The first, second, third, fourth and fifth cells 34, 36, 152, 192 and 194 can have first, second, third, fourth, and fifth cell angles 200, 202, 204, 206 and 208, respectively. The cell angle 234 can be the longitudinal-facing angle on the cell. The cell angles 234 can be from about 7.9° to about 21.27°, for example about 7.9° or about 8.0° or about 8.1 ° or about 9.9° or about 11.9° or about 15.9° or about 18.2° or about 21.27°.

The struts 220 can have strut thicknesses 210. The strut thicknesses 210 can be from about 0.53 mm (0.021 in.) to about 0.91 mm (0.036 in.), for example 0.53 mm (0.021 in.) or about 0.76 mm (0.030 in.) or about 0.91 mm (0.036 in.).

Figures 54 and 55 illustrate an embodiment of the expandable support device 14 similar to the expandable support device 14 shown in Figures 52 and 53, but with different exemplary dimensions.

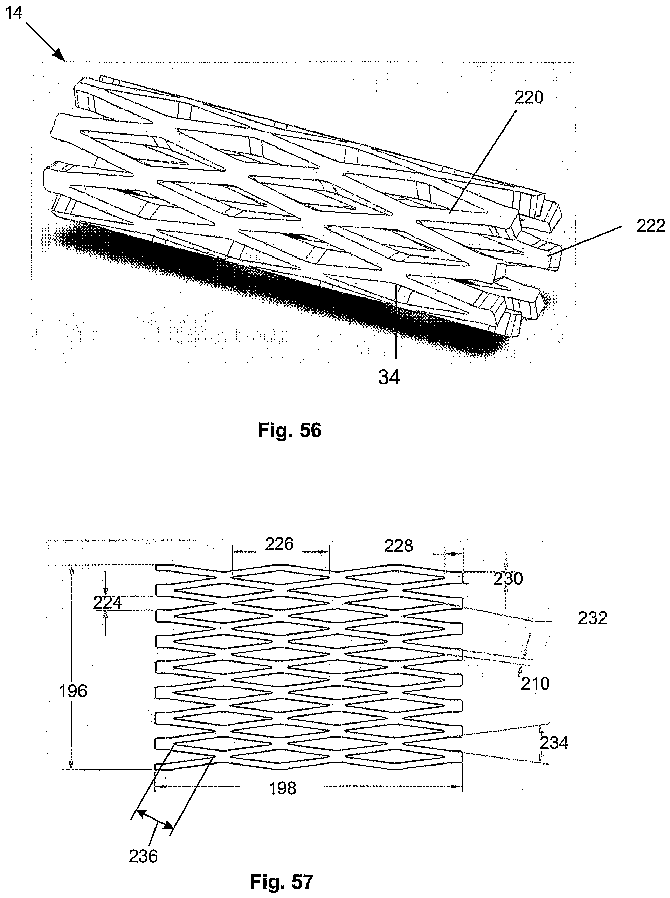

Figures 56 and 57 illustrate that the expandable support device 14 can have end tabs 222. The end tabs 222 can be blunt and/or sharpened. The expandable support device 14 can have no end rings 174. The struts 220 can have a constant strut thickness 210 along the expandable support device 14. The cells can have uniform dimensions along the expandable support device 14, when the expandable support device 14 is in a radially contracted configuration.

The cells can have a cell corner radius 232 (e.g., about 0.08 mm (0.003 in.)). The struts 220 can have strut lengths 236. The strut lengths 236 can be equal to the cell width 226. The strut length 236 can be about 3.78 mm (0.149 in.).

The end tabs 222 can have end tab widths 228 and end tab heights 230. The end tab width 228 can be about 1.6 mm (0.064 in.). The end tab height 230 can be about 1.4 mm (0.057 in.).

The cell can have a cell height 224. The cell height 224 can be the link height 182. The cell height 224 can be about 1.7 mm (0.067 in.).

Any or all elements of the expandable support device 14, deployment tool 76 and/or other devices or apparatuses described herein can be made from, for example, a single or multiple stainless steel alloys (e.g., 304 SS, annealed), titanium alloys (e.g., titanium G2), nickel titanium alloys (e.g., Nitinol), cobalt-chrome alloys (e.g., ELGILOY® from Elgin Specialty Metals, Elgin, IL; CONICHROME® from Carpenter Metals Corp., Wyomissing, PA), nickel-cobalt alloys (e.g., MP35N® from Magellan Industrial Trading Company, Inc., Westport, CT), molybdenum alloys (e.g., molybdenum TZM alloy, for example as disclosed in International Pub. No. WO 03/082363 A2, published 9 October 2003), tungsten-rhenium alloys, for example, as disclosed in International Pub. No. WO 03/082363, polymers such as polyethylene teraphathalate (PET), polyester (e.g., DACRON® from E. I. Du Pont de Nemours and Company, Wilmington, DE), polypropylene, aromatic polyesters, such as liquid crystal polymers (e.g., Vectran, from Kuraray Co., Ltd., Tokyo, Japan), ultra high molecular weight polyethylene (i.e., extended chain, high-modulus or high-performance polyethylene) fiber and/or yarn (e.g., SPECTRA® Fiber and SPECTRA® Guard, from Honeywell International, Inc., Morris Township, NJ, or DYNEEMA® from Royal DSM N.V., Heerlen, the Netherlands), polytetrafluoroethylene (PTFE), expanded PTFE (ePTFE), polyether ketone (PEK), polyether ether ketone (PEEK), poly ether ketone ketone (PEKK) (also poly aryl ether ketone ketone), nylon, polyether-block co-polyamide polymers (e.g., PEBAX® from ATOFINA, Paris, France), aliphatic polyether polyurethanes (e.g., TECOFLEX® from Thermedics Polymer Products, Wilmington, MA), polyvinyl chloride (PVC), polyurethane, thermoplastic, fluorinated ethylene propylene (FEP), absorbable or resorbable polymers such as polyglycolic acid (PGA), poly-L-glycolic acid (PLGA), polylactic acid (PLA), poly-L-lactic acid (PLLA), polycaprolactone (PCL), polyethyl acrylate (PEA), polydioxanone (PDS), and pseudo-polyamino tyrosine-based acids, extruded collagen, silicone, zinc, echogenic, radioactive, radiopaque materials, a biomaterial (e.g., cadaver tissue, collagen, allograft, autograft, xenograft, bone cement, morselized bone, osteogenic powder, beads of bone) any of the other materials listed herein or combinations thereof. Examples of radiopaque materials are barium sulfate, zinc oxide, titanium, stainless steel, nickel-titanium alloys, tantalum and gold.

Any or all elements of the expandable support device 14, deployment tool 76 and/or other devices or apparatuses described herein, can be, have, and/or be completely or partially coated with agents and/or a matrix a matrix for cell ingrowth or used with a fabric, for example a covering (not shown) that acts as a matrix for cell ingrowth. The matrix and/or fabric can be, for example, polyester (e.g., DACRON® from E. I. Du Pont de Nemours and Company, Wilmington, DE), polypropylene, PTFE, ePTFE, nylon, extruded collagen, silicone or combinations thereof.

The expandable support device 14, deployment tool 76 and/or elements of the expandable support device 14, deployment tool 76 and/or other devices or apparatuses described herein and/or the fabric can be filled, coated, layered and/or otherwise made with and/or from cements, fillers, glues, and/or an agent delivery matrix known to one having ordinary skill in the art and/or a therapeutic and/or diagnostic agent. Any of these cements and/or fillers and/or glues can be osteogenic and osteoinductive growth factors.

Examples of such cements and/or fillers includes bone chips, demineralized bone matrix (DBM), calcium sulfate, coralline hydroxyapatite, biocoral, tricalcium phosphate, calcium phosphate, polymethyl methacrylate (PMMA), biodegradable ceramics, bioactive glasses, hyaluronic acid, lactoferrin, bone morphogenic proteins (BMPs) such as recombinant human bone morphogenetic proteins (rhBMPs), other materials described herein, or combinations thereof.

The agents within these matrices can include any agent disclosed herein or combinations thereof, including radioactive materials; radiopaque materials; cytogenic agents; cytotoxic agents; cytostatic agents; thrombogenic agents, for example polyurethane, cellulose acetate polymer mixed with bismuth trioxide, and ethylene vinyl alcohol; lubricious, hydrophilic materials; phosphor cholene; anti-inflammatory agents, for example non-steroidal anti-inflammatories (NSAIDs) such as cyclooxygenase-1 (COX-1) inhibitors (e.g., acetylsalicylic acid, for example ASPIRIN® from Bayer AG, Leverkusen, Germany; ibuprofen, for example ADVIL® from Wyeth, Collegeville, PA; indomethacin; mefenamic acid), COX-2 inhibitors (e.g., VIOXX® from Merck & Co., Inc., Whitehouse Station, NJ; CELEBREX® from Pharmacia Corp., Peapack, NJ; COX-1 inhibitors); immunosuppressive agents, for example Sirolimus (RAPAMUNE®, from Wyeth, Collegeville, PA), or matrix metalloproteinase (MMP) inhibitors (e.g., tetracycline and tetracycline derivatives) that act early within the pathways of an inflammatory response. Examples of other agents are provided in Walton et al, Inhibition of Prostaglandin E2 Synthesis in Abdominal Aortic Aneurysms, Circulation, July 6, 1999, 48-54; Tambiah et al, Provocation of Experimental Aortic Inflammation Mediators and Chlamydia Pneumoniae, Brit. J. Surgery 88 (7), 935-940; Franklin et al, Uptake of Tetracycline by Aortic Aneurysm Wall and Its Effect on Inflammation and Proteolysis, Brit. J. Surgery 86 (6), 771-775; Xu et al, Sp1 Increases Expression of Cyclooxygenase-2 in Hypoxic Vascular Endothelium, J. Biological Chemistry 275 (32) 24583-24589; and Pyo et al, Targeted Gene Disruption of Matrix Metalloproteinase-9 (Gelatinase B) Suppresses Development of Experimental Abdominal Aortic Aneurysms, J. Clinical Investigation 105 (11), 1641-1649.

The expandable support device 14 can be any expandable support device 14 or combinations thereof or include elements thereof as described in PCT Application Numbers US2005/034,115, filed 21 September 2005; US2005/034,742, filed 26 September 2005; US2005/034,728 filed 26 September 2005, US2005/037,126, filed 12 October 2005; and U.S. Provisional Patent Application Numbers 60/612,001, filed 21 September 2004; 60/675,543, filed 27 April 2005; 60/612,723, filed 24 September 2004; 60/612,728, filed 24 September 2004; 60/617,810, filed 12 October 2004; 60/723,309, filed 4 October 2005; 60/741,201, filed 1 December 2005; 60/754,239, filed 27 December 2005; 60/741,197, filed 1 December 2005; 60/751,882, filed 19 December 2005; 60/675,512, filed 27 April 2005; 60/752,180, filed 19 December 2005; 60/699,577, filed 14 July 2005; 60/699,576 filed 14 July 2005; 60/754,492, filed 28 December 2005; 60/751,390 filed 15 December 2005; 60/752,186 19 filed December 2005; 60/754,377 filed 27 December 2005; 60/754,485, filed 28 December 2005; 60/754,227 filed 28 December 2005; 60/752,185 filed 19 December 2005; 60/735,718, filed 11 November 2005; 60/752,183, filed 19 December 2005; and 60/752,182, filed 19 December 2005.

### METHODS OF MANUFACTURE

The expandable support devices 14 can be hollow tubes before the cells are formed. The cells can be cut or slotted (e.g., laser-slotted or laser-cut) from the hollow tubes. The tubes can be hollowed before and/or after the cells are formed.

The expandable support devices 14 can be made from individual filaments in a braided configuration. The device can be locked open by controlling the expanded braid's length. The device can be made from coiled springs. The coiled springs can be twisted into a small diameter, then untwisted or plastically twisted open to create radial force. The devices can be made from any combination of the above methods.

### METHODS OF USE

Figure 58 illustrates that an expandable support device 14 can be deployed into a bone, such as a first vertebra 240. A deployment channel 238 can be drilled or otherwise formed in the first vertebra 240. The deployment channel 238 can provide access to a treatment site in the first vertebra 240. The deployment channel 238 can be formed by the expandable support device 14 during deployment (e.g., ramming and crushing, screwing and displacing, ultrasound shaking and disintegrating, or otherwise transmitting energy through the expandable support device 14 to form the deployment channel 238).

The expandable support device 14 can be inserted along the insertion path 246 into the first vertebra 240, for example from the caudal and/or dorsal 252 side of the vertebra. (Ventral 250 and dorsal 252 directions and the spinal cord 248 are shown for orientation.) When the expandable support device 14 is in the first vertebra 240, the expandable support device 14 can be longitudinally compressed, as shown by arrows 162. The longitudinal compression can cause the cells to flex such that the expandable support device 14 can radially expand, as shown by arrows 18.

Figure 59 illustrates that the expandable support device 14 can be deployed into a treatment site in an intervertebral disc 244. The intervertebral disc 244 can be partially or completely removed (e.g., by surgery or by a pathology) before the expandable support device 14 is inserted into the treatment site. The expandable support device 14 can be inserted along the insertion path 246, for example from a directly dorsal 252 location. When the expandable support device 14 is in the first vertebra 240, the expandable support device 14 can be longitudinally compressed, as shown by arrows 162. The longitudinal compression can cause the cells to flex such that the expandable support device 14 can radially expand, as shown by arrows 18. In a radially expanded configuration, the expandable support device 14 can be in contact with the first vertebra 240 and a second vertebra 242.

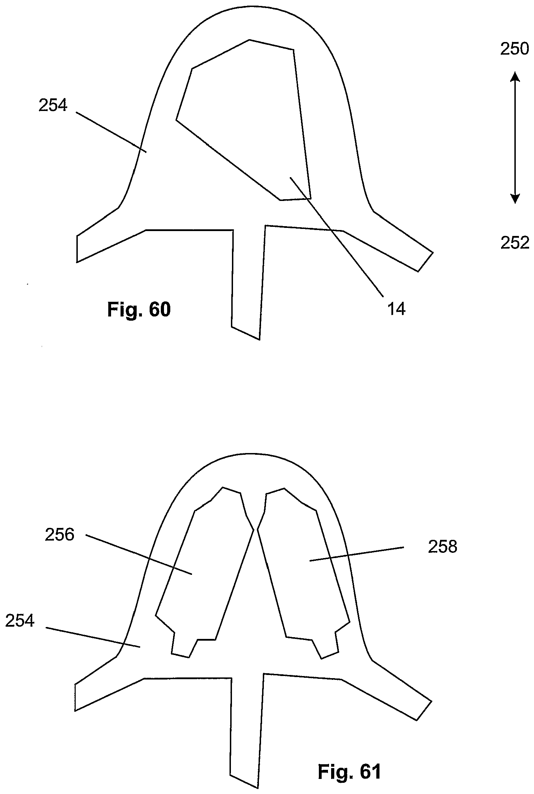

Figure 60 illustrates that a single expandable support device 14 can be deployed at an angle with respect to the center sagittal plane.

Figure 61 illustrates that a first expandable support device 256 can be deployed in a symmetrically offset (i.e., at the negative distance and angles) configuration with respect to the center sagittal plane compared to a second expandable support device 258.

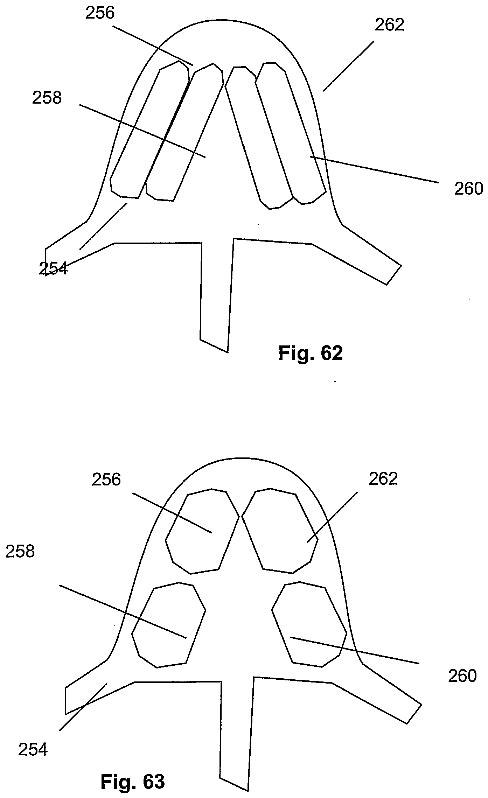

Figure 62 illustrates that the first and second expandable support devices 258 can be deployed on one side of the center sagittal plane symmetrically offset with respect to the center sagittal plane compared to the third and fourth expandable support devices 262. The first expandable support device 256 can be deployed parallel and adjacent (e.g., in contact and/or attached) to the second expandable support device 258. The third expandable support device 260 can be deployed parallel and adjacent (e.g., in contact and/or attached) to the fourth expandable support device 262.

Figure 63 illustrates that the first expandable support device 256 can be deployed on one side of the center sagittal plane symmetrically offset with respect to the center sagittal plane compared to the fourth expandable support device 262. The second expandable support device 258 can be deployed on one side of the center sagittal plane symmetrically offset with respect to the center sagittal plane compared to the third expandable support device 260. The first 256 and fourth 262 expandable support devices can be offset from the center dorsal 252 plane at the negative distance that the second 258 and third 260 expandable support devices are offset from the center dorsal 252 plane.

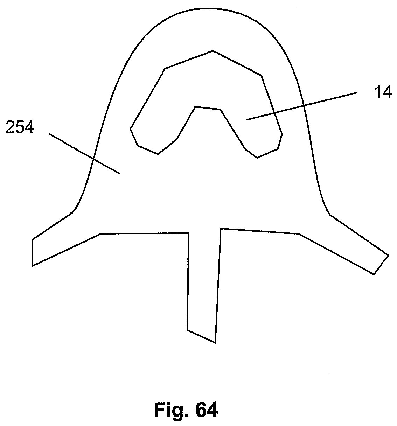

Figure 64 illustrates that the expandable support device 14 can be deployed in a curved or otherwise angled configuration.

The configurations illustrated in Figures 60 through 64 can be used for intervertebral and/or intravertebral deployment of the expandable support device 14. The deployed position of the expandable support device 14 is shown over the transverse (i.e., horizontal) cross-section of the vertebra 254. After deployment in a bone, the expandable support device 14 can be partially or completely filled with any of the materials disclosed herein, including BMPs, morselized bone, DBM, and combinations thereof.

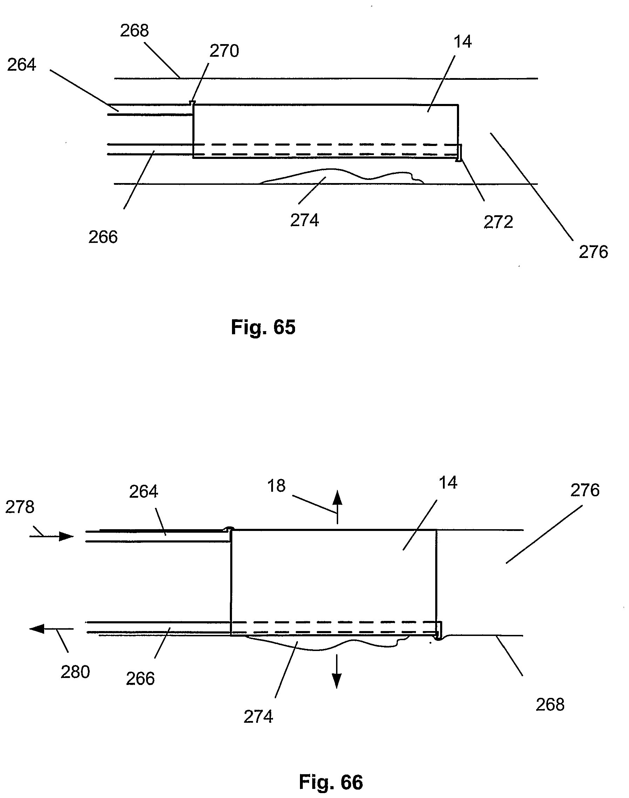

Figure 65 illustrates that the expandable support device 14 can be loaded onto a deployment tool 76 and positioned, as shown by arrow, into a lumen 276 at or adjacent to a treatment site. The lumen 276 can be a venous or arterial blood vessel (e.g., coronary or peripheral). The lumen 276 can have a damaged lumen wall 274 at the treatment site, for example as a symptom of atherosclerosis (e.g., stenosis).

The first deployment arm 264 can have a first catch 270. The second deployment arm 266 can have a second catch 272. The first catch 270 can be removably attached to the first end of the expandable support device 14. The second catch 272 can be removably attached to the second end of the expandable support device 14.

Figure 66 illustrates that a first translating force 278, as shown by arrow, can be applied to the first deployment arm 264 and that a substantially equal and opposite second translating force 280, as shown by arrow, can be applied to the second deployment arm 266. The translating forces can be transmitted to longitudinally compress the expandable support device 14. The expandable support device 14 can radially expand, as shown by arrows. The expanded expandable support device 14 can treat damaged the treatment site, for example by reconfiguring the lumen wall 268. The first 270 and second catches 272 can be removed from the expandable support device 14. The deployment tool 76 can be removed from the treatment site.

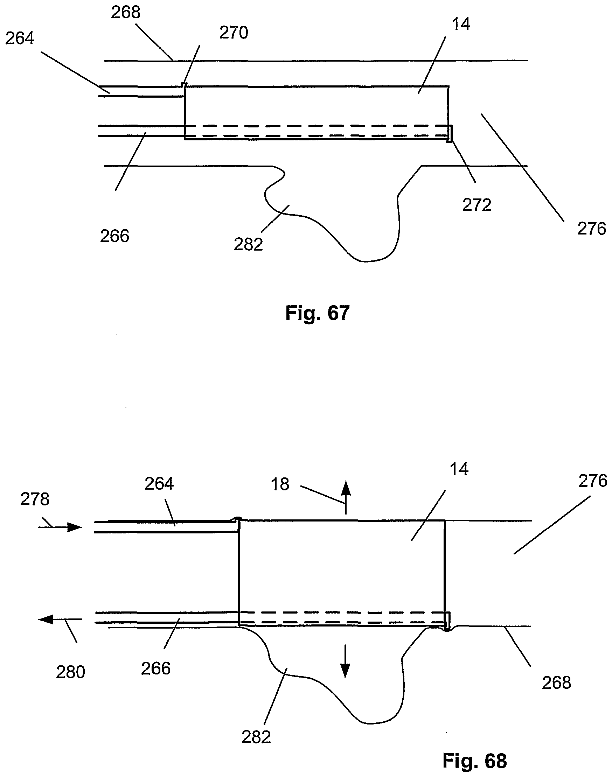

Figure 67 illustrates that the treatment site can be an aneurysm 282. The aneurysm 282 can be thoracic, abdominal, cerebral, coronary, or other vascular aneurysms. The aneurysm 282 can be fusiform, saccular, a pseudoaneurysm, or combinations thereof. Figure 68 illustrates that the expandable support device 14 can be radially expanded as shown in Figure 66. The expandable support device 14 can cover the aneurysm neck, for example minimizing and/or preventing fluid flow 296 into and/or out of the aneurysm 282. The expandable support device 14 can have a coating and/or a graft minimizing and/or preventing fluid flow 296 through the wall of the expandable support device 14.

One or more expandable support devices 14 with or without grafts can be deployed into the aneurysm 282, for example before the expandable support device 14 is deployed as shown in Figure 68. The expandable support devices 14 can be radially expanded or not radially expanded.

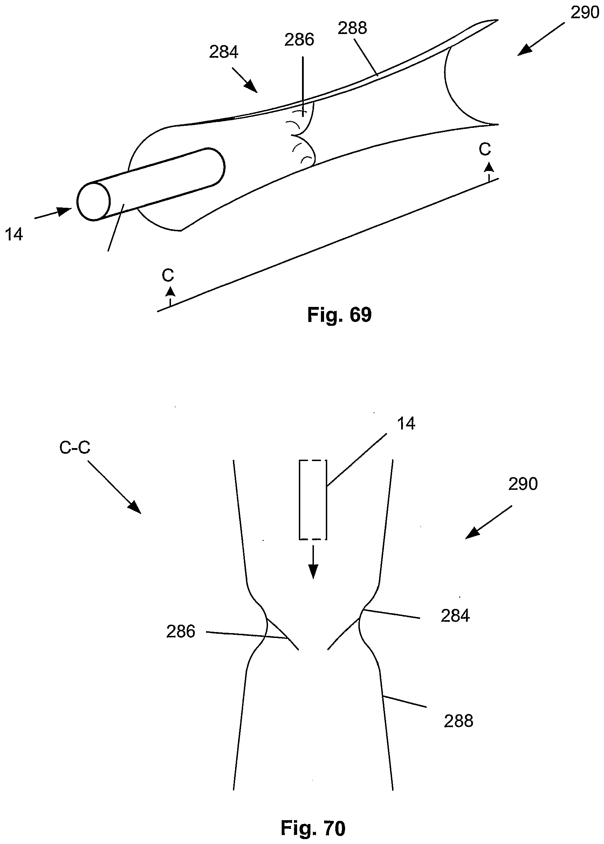

Figures 69 and 70 illustrate that the expandable support device 14 can positioned, as shown by arrow, at or adjacent to a valve 290. The valve 290 can have a valvular lumen wall 288. The valve 290 can have an annulus 284 and/or leaflets 286. The valve 290 can be a heart valve, for example a stenotic mitral or aortic valve.

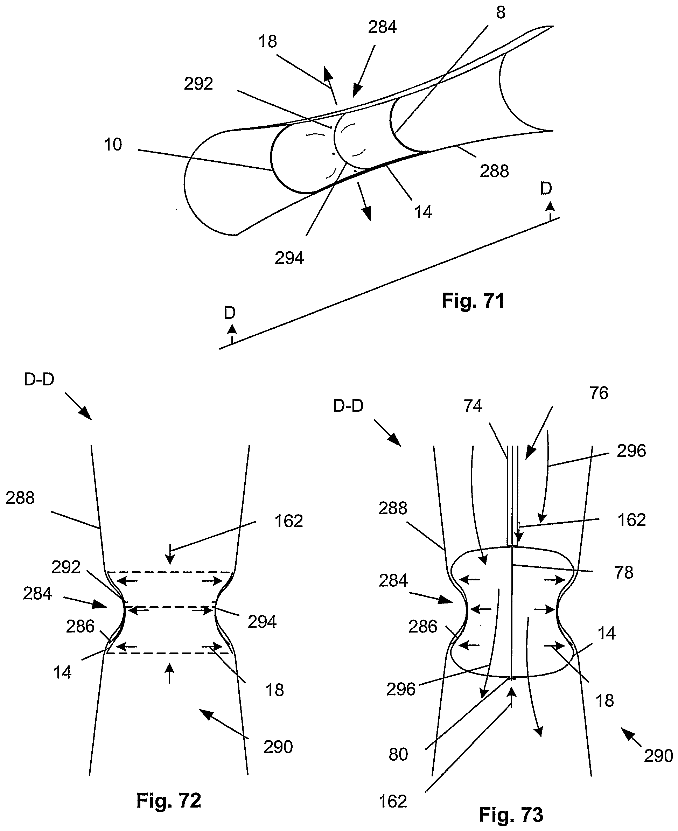

As shown in Figure 71, the expandable support device 14 can be positioned to longitudinally overlap the annulus 284 and/or leaflets 286. The expandable support device 14 can then be longitudinally compressed, for example causing radial expansion 18. The expandable support device 14 can have attachment devices, such as attachment tabs 292 and/or an expandable rim 294, for attaching new leaflets 286. The expandable rim 294 can circumferentially lock during deployment.

As shown in Figures 72 and 73, the expandable support device 14 can be longitudinally compressed, for example causing radial expansion 18 of the expandable support device 14. The cells of the expandable support device 14 can be configured to taper the middle of the expandable support device 14 during radial expansion 18, such as shown in Figures 71, 72 and 73. For example, the cells at or near the ends of the expandable support device 14 can be configured to radially expand more than the cells at or near the middle of the expandable support device 14. Also for example, the expandable support device 14 can be deformable and a balloon as shown in Figures 9 or 10 of PCT Application Number US2005/033,965, filed 21 September 2005; and U.S. Provisional Patent Application Numbers 60/611,972, filed 21 September 2004 published as WO 2006/034396, can be used to radially expand the expandable support device 14. U.S. Provisional Patent Application Number 60/740,792 filed 30 November 2005 published as WO 2007/065137.

The first end and/or second end of the expandable support device 14 can be completely or substantially open, as shown in Figures 71 and 72, for example, allowing flow through the valve 290 during deployment of the expandable support device 14. The first end and/or second end of the expandable support device 14 can have cells, as shown in Figure 73, for example, allowing flow through the valve 290, as shown by arrows, during deployment of the expandable support device 14.

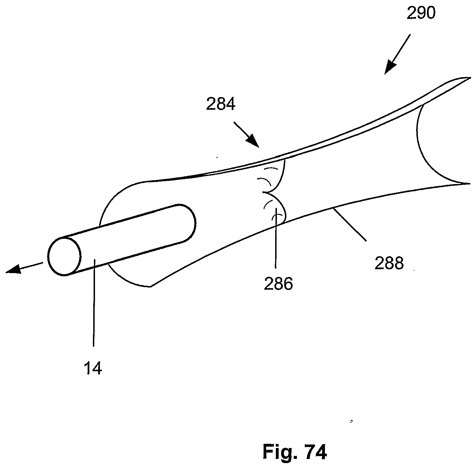

Figure 74 illustrates that the expandable support device 14 can be radially contracted (e.g., by applying a longitudinal tensile force, and/or a radial tensile force, and/or removing the compressive forces 6 on a radially self-contracting expandable support device 14). The expandable support device 14 can be withdrawn from the valve 290, as shown by arrow.

The expandable support device 14 can be left in the valve 290 permanently or semi-permanently. New leaflets 286 can be attached to the expandable support device 14, for example at the attachment tabs 292 and/or expandable rim 294.

Figures 69, 70, 71, 72 and 74 do not show the deployment tool 76 for clarity of illustration.

The expandable support device 14 can be used for tissue distraction.

One or more expandable support devices 14 can be used on one application (i.e., patient).

The deployment tools 76 used herein can, for example, be the deployment tools 76 disclosed herein, the deployment tool 76 (i.e., including balloons) disclosed in U.S. Provisional Patent Application Nos. 60/611,972 filed 21 September 2004 published as WO 2006/034396, 60/612,724 filed 24 September 2004 published as US 2007/219634, and 60/617,810 filed 12 October 2004 published as US 2007/219634.

It is apparent to one skilled in the art that various changes and modifications can be made to this disclosure, and equivalents employed, without departing from the scope of the claims. Elements shown with any embodiment are exemplary for the specific embodiment and can be used on or in combination with other embodiments within this disclosure. The devices, apparatuses and systems disclosed herein can be used for medical or non-medical, industrial applications.

## Claims

1. An expandable support device (14) for orthopedic treatment comprising:
a first end ring (106, 108, 174) having a first end ring opening, wherein the first end ring is at a first terminal end of the device;
a plurality of links (20), wherein at least some of the links (20) are attached to at least one other link at a joint (22, 24), and wherein at least some of the links (20) and joints (22, 24) form cells (2, 34, 36, 152, 192,194); and
wherein the device (14) has a longitudinal axis (12), and wherein the device (14) has a first configuration and a second configuration, and wherein when the device (14) is in the first configuration the device (14) is radially contracted about the longitudinal axis (12) and has a first length, and when the device (14) is in the second configuration a first longitudinal end (8) of the device and a second longitudinal end (10) of the device (14) are radially contracted about the longitudinal axis (12), and wherein the device (14) has a second length when the device (14) is in the second configuration; and
wherein the plurality of links (20) comprise a first link (20) and a second link (20) attached to the first link (20), and wherein the first link (20) has a first link angle (54) with respect to the longitudinal axis (12) of the device (14), and wherein the first link angle (54) is greater than zero and less than perpendicular when the device (14) is in the first configuration; and
wherein the first link (20) joins the second link (20) at a first joint (22, 24), and wherein a third link (20) joins a fourth link (20) at a second joint (22, 24), and wherein the third link (20) attaches to the first link (20) at a third joint (22, 24), and wherein the fourth link (20) attaches to the second link (20) at a fourth joint (22, 24), wherein a joint line between the first joint (22, 24) and the second joint (22, 24) in the first configuration is parallel with the longitudinal axis (12); and
wherein a middle length of the device (14) between the first longitudinal end of the device (14) and the second longitudinal end of the device (14) is more radially expanded (18) about the longitudinal axis (12) than the first longitudinal end (8) and the second longitudinal end (10) of the device (14), and wherein the first longitudinal end (8) comprises a cell (2, 34, 36, 152, 192,194).

2. The device (14) of Claim 1, wherein the links (20) comprise a metal.

3. The device (14) of Claim 1, wherein the links (20) comprise a polymer.

4. The device (14) of Claim 1, wherein at least one cell (2, 34, 36, 152, 192,194) is diamond shaped.

5. The device (14) of Claim 1, wherein at least one cell (2, 34, 36, 152, 192,194) is elongated compared to at least one other cell (2, 34, 36).

6. The device (14) of Claim 1, wherein when the device (14) is in the first configuration, the first link angle (54) is greater than about 7.9°.

7. The device (14) of Claim 1, wherein the device (14) in the second configuration has a curved outer surface.

8. The device (14) of Claim 1, wherein the second link (20) is configured to remain at a constant radius from the longitudinal axis (12) along the second link (20) when the device (14) transforms from the first configuration to the second configuration.

9. The device (14) of Claim 1, wherein the second length is shorter than the first length.

10. The device (14) of Claim 1, further comprising a longitudinal compression element (76) configured to longitudinally compress the plurality of links (20).

11. The device (14) of Claim 1, wherein the first link (20) has a constant radius from the longitudinal axis (12) along the length of the first link (20) when the device (14) is in the second configuration.

12. The device (14) of Claim 1, wherein the second link (20) is configured to rotate with respect to the first link (20) and when the device (14) transforms from the first configuration to the second configuration

13. The device (14) of Claim 1, wherein a first cell angle (200) is from about 7.9° to about 21.27° when the device (14) is in the first configuration.

14. The device (14) of Claim 1, wherein the first link (20) is not parallel with the second link (20) when the device (14) is in the first configuration.

15. The device (14) of Claim 1, further comprising a second end ring (106, 108, 174) having a second end ring opening, wherein the second end ring is at a second terminal end of the device, wherein at least some of the links (20) terminate at the first end ring, and wherein at least some of the links (20) terminate at the second end ring.

## Patentansprüche

1. Expandierbare Stützvorrichtung (14) für eine orthopädische Behandlung, Folgendes umfassend:
einen ersten Endring (106, 108, 174), der eine erste Endringöffnung aufweist, wobei der erste Endring sich an einem ersten Abschlussende der Vorrichtung befindet;
eine Vielzahl von Gliedern (20), wobei mindestens einige der Glieder (20) mindestens an einem anderen Glied an einem Gelenk (22, 24) befestigt sind und wobei mindestens einige der Glieder (20) und Gelenke (22, 24) Zellen (2, 34, 36, 152, 192, 194) bilden; und
wobei die Vorrichtung (14) eine Längsachse (12) aufweist und wobei die Vorrichtung (14) eine erste Auslegung und eine zweite Auslegung aufweist und wobei, wenn sich die Vorrichtung (14) in der ersten Auslegung befindet, die Vorrichtung (14) um die Längsachse (12) radial kontrahiert ist und eine erste Länge aufweist, und wenn die Vorrichtung (14) sich in der zweiten Auslegung befindet, ein erstes Längsende (8) der Vorrichtung und ein zweites Längsende (10) der Vorrichtung (14) um die Längsachse (12) radial kontrahiert sind, und wobei die Vorrichtung (14) eine zweite Länge aufweist, wenn die Vorrichtung (14) sich in der zweiten Auslegung befindet; und
wobei die Vielzahl von Gliedern (20) ein erstes Glied (20) umfasst und ein zweites Glied (20) das am ersten Glied (20) befestigt ist, und wobei das erste Glied (20) einen ersten Gliedwinkel (54) mit Bezug auf die Längsachse (12) der Vorrichtung (14) aufweist und wobei der erste Gliedwinkel (54) größer als null ist und kleiner als rechtwinklig, wenn sich die Vorrichtung (14) in der ersten Auslegung befindet; und
wobei das erste Glied (20) an einem ersten Punkt (22, 24) mit dem zweiten Glied (20) verbunden ist und wobei ein drittes Glied (20) an einem zweiten Punkt (22, 24) mit einem vierten Glied (20) verbunden ist und wobei das dritte Glied (20) an einem dritten Gelenk (22, 24) am ersten Glied (20) befestigt ist und wobei das vierte Glied (20) an einem vierten Gelenk (22, 24) am zweiten Glied (20) befestigt ist, wobei eine Gelenklinie zwischen dem ersten Gelenk (22, 24) und dem zweiten Gelenk (22, 24) in der ersten Auslegung parallel zur Längsachse (12) verläuft; und
wobei eine mittlere Länge der Vorrichtung (14) zwischen dem ersten Längsende der Vorrichtung (14) und dem zweiten Längsende der Vorrichtung (14) radialer um die Längsachse (12) expandiert ist (18) als das erste Längsende (8) und das zweite Längsende (10) der Vorrichtung (14) und wobei das erste Längsende (8) eine Zelle (2, 34, 36, 152, 192, 194) umfasst.

2. Vorrichtung (14) nach Anspruch 1, wobei die Glieder (20) ein Metall umfassen.

3. Vorrichtung (14) nach Anspruch 1, wobei die Glieder (20) ein Polymer umfassen.

4. Vorrichtung (14) nach Anspruch 1, wobei mindestens eine Zelle (2, 34, 36, 152, 192, 194) rautenförmig ist.

5. Vorrichtung (14) nach Anspruch 1, wobei mindestens eine Zelle (2, 34, 36, 152, 192,194) verglichen mit mindestens einer anderen Zelle (2, 34, 36) langgestreckt ist.

6. Vorrichtung (14) nach Anspruch 1, wobei, wenn die Vorrichtung (14) sich in der ersten Auslegung befindet, der erste Gelenkwinkel (54) größer ist als ungefähr 7,9°.

7. Vorrichtung (14) nach Anspruch 1, wobei die Vorrichtung (14) in der zweiten Auslegung eine gekrümmte Außenfläche aufweist.

8. Vorrichtung (14) nach Anspruch 1, wobei das zweite Glied (20) dazu ausgelegt ist, von der Längsachse (12) entlang des zweiten Glieds (20) in einem konstanten Radius zu verbleiben, wenn die Vorrichtung (14) von der ersten Auslegung zur zweiten Auslegung wechselt.

9. Vorrichtung (14) nach Anspruch 1, wobei die zweite Länge kürzer ist als die erste Länge.

10. Vorrichtung (14) nach Anspruch 1, die ferner ein Längskomprimierungselement (76) umfasst, das dazu ausgelegt ist, die Vielzahl von Gliedern (20) längs zu komprimieren.

11. Vorrichtung (14) nach Anspruch 1, wobei das zweite Glied (20) von der Längsachse (12) entlang der Länge des ersten Glieds (20) einen konstanten Radius aufweist, wenn die Vorrichtung (14) sich in der zweiten Auslegung befindet.

12. Vorrichtung (14) nach Anspruch 1, wobei das zweite Glied (20) dazu ausgelegt ist, sich mit Bezug auf das erste Glied (20) zu drehen, und wenn die Vorrichtung (14) von der ersten Auslegung zur zweiten Auslegung wechselt.

13. Vorrichtung (14) nach Anspruch 1, wobei ein erster Zellwinkel (200) von ungefähr 7,9° bis ungefähr 21,27° beträgt, wenn die Vorrichtung (14) sich in der ersten Auslegung befindet.

14. Vorrichtung (14) nach Anspruch 16, wobei das erste Glied (20) nicht parallel zum zweiten Glied (20) verläuft, wenn die Vorrichtung (14) sich in der ersten Auslegung befindet.

15. Vorrichtung (14) nach Anspruch 1, die ferner einen zweiten Endring (106, 108, 174) umfasst, der eine zweite Endringöffnung aufweist, wobei der zweite Endring sich an einem zweiten Abschlussende der Vorrichtung befindet, wobei mindestens einige der Glieder (20) am ersten Endring enden und wobei mindestens einige der Glieder (20) am zweiten Endring enden.

## Revendications

1. Dispositif support expansible (14) destiné à un traitement orthopédique comprenant :
une première bague d'extrémité (106, 108, 174) présentant une ouverture de première bague d'extrémité, la première bague d'extrémité étant située à une première extrémité terminale du dispositif ;
une pluralité de liaisons (20), au moins certaines de ces liaisons (20) étant attachées à au moins une autre liaison au niveau d'une articulation (22, 24), et au moins certaines de ces liaisons (20) et articulations (22, 24) formant des cellules (2, 34, 36, 152, 192, 194) ; et
le dispositif (14) présentant un axe longitudinal (12), et le dispositif (14) présentant une première configuration et une deuxième configuration, et lorsque le dispositif (14) est dans une première configuration, le dispositif (14) étant contracté radialement autour de l'axe longitudinal (12) et présentant une première longueur, et lorsque le dispositif (14) est dans la deuxième configuration, une première extrémité longitudinale (8) du dispositif et une deuxième extrémité longitudinale (10) du dispositif (14) étant contractées radialement autour de l'axe longitudinal (12), et le dispositif (14) présentant une deuxième longueur lorsque le dispositif (14) est dans une deuxième configuration ; et
la pluralité de liaisons (20) comprenant une première liaison (20) et une deuxième liaison (20) attachée à la première liaison (20), et la première liaison (20) présentant un premier angle de liaison (54) par rapport à l'axe longitudinal (12) du dispositif (14), et le premier angle de liaison (54) étant supérieur à zéro et de moins de 90° lorsque le dispositif (14) est dans la première configuration ; et
la première liaison (20) reliant la deuxième liaison (20) au niveau d'une première articulation (22, 24), et une troisième liaison (20) reliant une quatrième liaison (20) au niveau d'une deuxième articulation (22, 24), et la troisième liaison (20) s'attachant à la première liaison (20) au niveau d'une troisième articulation (22, 24), et la quatrième liaison (20) s'attachant à la deuxième liaison (20) au niveau d'une quatrième articulation (22, 24), une ligne de jonction entre la première articulation (22, 24) et la deuxième articulation (22, 24) dans la première configuration étant parallèle à l'axe longitudinal (12) ; et
une longueur médiane du dispositif (14) entre la première extrémité longitudinale du dispositif (14) et la deuxième extrémité longitudinale du dispositif (14) étant dilatée plus radialement (18) autour de l'axe longitudinal (12) que la première extrémité longitudinale (8) et la deuxième extrémité longitudinale (10) du dispositif (14), et la première extrémité longitudinale (8) comprenant une cellule (2, 34, 36, 152, 192, 194).

2. Dispositif (14) selon la revendication 1, dans lequel les liaisons (20) comprennent un métal.

3. Dispositif (14) selon la revendication 1, dans lequel les liaisons (20) comprennent un polymère.

4. Dispositif (14) selon la revendication 1, dans lequel au moins une cellule (2, 34, 36, 152, 192, 194) est en forme de diamant.

5. Dispositif (14) selon la revendication 1, dans lequel au moins une cellule (2, 34, 36, 152, 192, 194) est oblongue comparée à au moins une autre cellule (2, 34, 36).

6. Dispositif (14) selon la revendication 1, dans lequel lorsque le dispositif (14) est dans la première configuration, le premier angle de liaison (54) est supérieur à environ 7,9°.

7. Dispositif (14) selon la revendication 1, dans lequel le dispositif (14) dans la deuxième configuration présente une surface externe incurvée.

8. Dispositif (14) selon la revendication 1, dans lequel la deuxième liaison (20) est conçue pour demeurer à un rayon constant à partir de l'axe longitudinal (12) le long de la deuxième liaison (20) lorsque le dispositif (14) se transforme de la première configuration en deuxième configuration.

9. Dispositif (14) selon la revendication 1, dans lequel la deuxième longueur est plus courte que la première longueur.

10. Dispositif (14) selon la revendication 1, comprenant en outre un élément de compression longitudinale (76) conçu pour comprimer longitudinalement la pluralité de liaisons (20).

11. Dispositif (14) selon la revendication 1, dans lequel la première liaison (20) présente un rayon constant à partir de l'axe longitudinal (12) sur la longueur de la première liaison (20) lorsque le dispositif (14) est dans la deuxième configuration.

12. Dispositif (14) selon la revendication 1, dans lequel la deuxième liaison (20) est conçue pour tourner par rapport à la première liaison (20) et lorsque le dispositif (14) se transforme de la première configuration en deuxième configuration.

13. Dispositif (14) selon la revendication 1, dans lequel un premier angle de cellule (200) va d'environ 7,9° à environ 21,27° lorsque le dispositif (14) est dans la première configuration.

14. Dispositif (14) selon la revendication 1, dans lequel la première liaison (20) n'est pas parallèle à la deuxième liaison (20) lorsque le dispositif (14) est dans la première configuration.

15. Dispositif (14) selon la revendication 1, comprenant en outre une deuxième bague d'extrémité (106, 108, 174) présentant une ouverture de deuxième bague d'extrémité, la deuxième bague d'extrémité étant située à une deuxième extrémité terminale du dispositif, au moins certaines des liaisons (20) terminant à la première bague d'extrémité et au moins certaines des liaisons (20) se terminant à la deuxième bague d'extrémité.
